# EUROPÄISCHE PATENTSCHRIFT

(11) **EP 2 624 947 B1**
(45) Veröffentlichungstag und Bekanntmachung des Hinweises auf die Patenterteilung: **27.04.2016**
(21) Anmeldenummer: 10760730.1
(22) Anmeldetag: 06.10.2010
(51) Int. Cl.: B01J 8/06, C07C 29/152

(54) **VERFAHREN UND ANLAGE ZUR SYNTHESE VON KOHLENWASSERSTOFF**
METHOD AND INSTALLATION FOR SYNTHESISING HYDROCARBON
PROCÉDÉ ET INSTALLATION DE SYNTHÈSE D'HYDROCARBURE

(43) Veröffentlichungstag der Anmeldung: 14.08.2013
(73) Patentinhaber: Silicon Fire AG, 6045 Meggen (CH)
(72) Erfinder: BREHM, Lothar, 61138 Niederdorfelden (DE)
(74) Vertreter: Heusch, Christian
(86) Internationale Anmeldenummer: PCT/EP2010/064948
(87) Internationale Veröffentlichungsnummer: WO 2012/045349

(56) Entgegenhaltungen:
- DE-A1- 3 442 053
- DE-C- 933 087
- US-A1- 2010 068 128

## Beschreibung

Die vorliegende Anmeldung betrifft Verfahren und Anlagen zum Bereitstellen speicherbarer und transportabler kohlenwasserstoffhaltiger Energieträger. Insbesondere geht es um die Methanolsynthese.

Kohlenstoffdioxid CO₂ (meist Kohlendioxid genannt) ist eine chemische Verbindung aus Kohlenstoff und Sauerstoff. Kohlendioxid ist ein farb- und geruchloses Gas. Es ist mit einer geringen Konzentration ein natürlicher Bestandteil der Luft und entsteht in Lebewesen bei der Zellatmung, aber auch bei der Verbrennung von kohlenstoffhaltigen Substanzen bei ausreichender Anwesenheit von Sauerstoff. Seit Beginn der Industrialisierung steigt der CO₂-Anteil in der Atmosphäre deutlich an. Hauptursache hierfür sind die vom Menschen verursachten - sogenannten anthropogenen - CO₂-Emissionen. Das Kohlendioxid in der Atmosphäre absorbiert einen Teil der Wärmestrahlung. Diese Eigenschaft macht Kohlendioxid zu einem so genannten Treibhausgas (THG) und zu einem der Mitverursacher des globalen Treibhauseffekts.

Aus diesen und auch aus anderen Gründen wird zur Zeit in verschiedenste Richtungen geforscht und entwickelt, um einen Weg zu finden, um die anthropogenen CO₂-Emissionen zu reduzieren. Besonders im Zusammenhang mit der Energieerzeugung, die häufig durch das Verbrennen fossiler Energieträger, wie Kohle, Öl oder Gas, erfolgt, aber auch mit anderen Verbrennungsprozessen, zum Beispiel der Müllverbrennung, besteht ein großer Bedarf zur Reduktion der CO₂-Emission. Es werden pro Jahr über 20 Milliarden Tonnen CO₂ durch solche Prozesse in die Atmosphäre abgegeben.

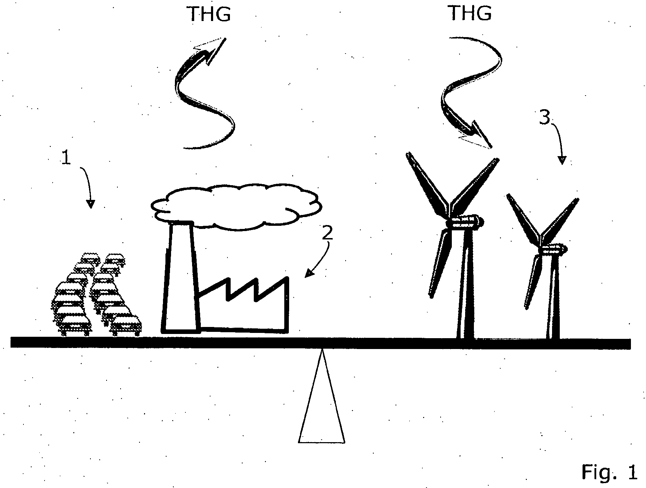

Es wird unter anderem das Prinzip der Klimaneutralität angestrebt, indem Ansätze verfolgt werden, bei denen man versucht, die mit CO₂-Emissionen verbundene Energieerzeugung an einem Ort durch das Erzeugen alternativer Energien an anderen Orten zu kompensieren. Dieser Ansatz ist stark schematisiert in Fig. 1 dargestellt. Emittenten von Treibhausgasen (THG), wie Industrieunternehmen (z.B. Automobilhersteller) 1 oder Kraftwerksbetreiber 2, investieren oder betreiben z.B. Windfarmen 3 an anderen Standorten im Rahmen von Ausgleichsprojekten, um dort Energie ohne THG- Emissionen zu erzeugen. Rein rechnerisch kann sich damit eine Klimaneutralität ergeben. Zahlreiche Firmen versuchen, sich auf diesem Weg eine "klimaneutrale Weste" zu erkaufen.

Es wird als Problem angesehen, dass zur Zeit nahezu alle regenerative elektrische Energie, die erzeugt wird, in das öffentliche Wechselspannungs-Verbundnetz eingespeist wird, dessen Frequenz nur innerhalb sehr enger Grenzen schwanken darf (z.B. +/- 0,4 %). Das kann nur erreicht werden, wenn die Stromerzeugung im Netz praktisch immer gleich dem Verbrauch ist. Die Notwendigkeit, für Wind- und Solarkraftwerke immer die ausreichenden Reserve- und Frequenzregelkapazitäten vorhalten zu müssen, führt zur entsprechenden Verteuerung der Stromversorgung mit diesen Anlagen. Wind- und Solarkraftwerke im elektrischen Verbundnetz ziehen somit weitere "versteckte" Kosten und Probleme nach sich.

Bereits beim heutigen Ausbaustand von Windkraftwerken kann in vielen Ländern das elektrische Energieversorgungsnetz vor ernste Probleme gestellt werden, wenn z.B. infolge von Windmangel oder Starkwind die Windleistung in großem Umfang ausfällt, vor allem wenn dieser Ausfall plötzlich und unerwartet erfolgt. In jedem Fall sind aber der installierten Wind- und Solarleistung angepasste Reserve- und Frequenzregelkapazitäten notwendig.

Daraus folgt, dass Solar- und Windkraftwerke, die in ein elektrisches Verbundnetz einspeisen, kaum die installierten Leistungen anderer Kraftwerke im Verbundnetz ersetzen können. Das führt dazu, dass Solar- und Windstrom in etwa nur mit den ersparten Brennstoffkosten der anderen, im Netz vorhandenen Wärmekraftwerke bewertet werden kann.

Es wurde bereits gezeigt, dass sich die regenerativen Energieformen besonders vorteilhaft mit fossilen Energieformen kombinieren lassen. Details hierzu sind zum Beispiel den folgenden Parallelanmeldungen des vorliegenden Anmelders zu entnehmen: publizierte internationale Anmeldung WO2010069385A1; publizierte internationale Anmeldung WO2010069622A1; europäische Patentanmeldung EP 10155530.8 (EP 2 226 290).

Eine solche Kombination ermöglicht es, kohlenwasserstoff-basierte Energieträger in entsprechenden Silicon-Fire Anlagen herzustellen. Diese Silicon-Fire Anlagen sind besonders geeignet, um damit Methanol herzustellen.

Es sind zahlreiche Verfahren und Reaktoren zur Herstellung von Methanol bekannt. Im Folgenden sind entsprechende beispielhafte Patentanmeldungen und Patente genannt:
- EP 0 790 226 B1;
- WO 2010/037441 A1;
- EP 0 483 919 A2;
- US 2010/0068128 A1;
- DE 34 42 053 A1; und
- DE 933 087

Beispielsweise offenbart US 2010/0068128 A1 ein Verfahren und einen Reaktor zum kontinuierlichen Herstellen von Schwefelwasserstoff (H₂S). Der Reaktor umfasst einen zylindrischen Hauptteil, der von einer oberen und einer unteren Haube abgeschlossen wird. Der Hauptteil enthält eine in einem oberen Bereich angeordnete Sammelwanne mit einem Produktdurchlass, einem Einlassstutzen, und einem nach unten führenden Rückführungsrohr, eine unterhalb der Sammelwanne angeordnete Trennplatte, die den Hauptteil unterteilt in einen oberen Teilbereich oberhalb der Trennplatte und in einen unteren Teilbereich unterhalb der Trennplatte, und eine unten im unteren Teilbereich angeordnete Verteilerplatte mit einer Vielzahl von Durchlassöffnungen zum Durchlassen von Wasserstoffgas. Der Reaktor umfasst ferner einen Schwefeleinlass zum Einlassen von flüssigem Schwefel und zum teilweise Befüllen des unteren Teilbereichs des Hauptteils mit einer Schwefelschmelze. Die Schwefelschmelze ist über eine Phasengrenze in Kontakt mit einem Reaktantenbereich, der an seiner Oberseite durch die Trennplatte begrenzt ist und im Betrieb des Reaktors hauptsächlich Wasserstoffgas und Schwefel enthält. In dem unteren Teilbereich des Hauptteils ist eine Vielzahl von parallel zueinander geschalteten U-förmigen Rohren angeordnet, die jeweils zwei gegenläufige angeordnete Schenkel und ein die beiden Schenkel an ihren jeweiligen unteren Enden strömungstechnisch verbindendes 180-Grad-Umlenkelement aufweisen. In jedem Schenkel ist ein festes Katalysatorbett angeordnet. Die Schenkel sind teilweise in die Schwefelschmelze eingetaucht, was einen Wämeaustausch zwischen dem Innenraum der Schenkel und der Schwefelschmelze ermöglicht. Eine Einlassöffnung zum Einlassen der Reaktantenmischung in den ersten abwärtsleitenden Schenkel ist im Reaktantenbereich in der Rohrwand des Schenkels vorgesehen. Das Ende des zweiten aufwärtsleitenden Schenkels ist oberhalb der Trennplatte in dem oberen Teilbereich des Hauptteils angeordnet und bildet eine Auslassöffnung. Der Reaktor umfasst ferner eine Zuführeinrichtung mit einer Einlassleitung und einem Rohr zum Zuführen von Wasserstoff in gasförmiger Form. Der Auslass des Rohres befindet sich im unteren Teilbereich des Hauptteils, unterhalb der Verteilerplatte.

Im Betrieb des Reaktors aus der US 2010/0068128 A1 wird zunächst die Schwefelschmelze durch den Schwefeleinlass eingefüllt, bis deren Phasengrenze in dem unteren Teilbereich des Hauptteils bis kurz unterhalb der Einlassöffnung des abwärtsführenden Schenkels angestiegen ist. Dann wird kontinuierlich Wasserstoffgas durch die Zuführeinrichtung und das Rohr eingelassen. Das Wasserstoffgas sammelt sich unter der Verteilerplatte, bildet dort eine Wasserstoffblase, aus der heraus durch die Durchlassöffnungen in der Verteilerplatte Gasbläschen in die und durch die Schwefelschmelze aufsteigen bis in den Reaktantenbereich und dabei Schwefel aus der Schwefelschmelze mitreißen, wodurch sich im Reaktantenbereich eine Reaktantenmischung aus gasförmigem Wasserstoff und Schwefel ansammelt. Diese Reaktantenmischung tritt durch die Einlassöffnungen in das Innere des Schenkels ein, strömt durch das Katalysatorbett zunächst abwärts in dem abwärtsführenden Schenkel, durch das Umlenkelement, dann weiter durch das Katalysatorbett aufwärts in dem aufwärtsführenden Schenkel und durch dessen Auslassöffnung in den Produktbereich, der die Sammelwanne enthält. In dem Produktbereich sammelt sich das Produkt und kann durch die obere Haube abgegeben werden.

DE 34 42 053 A1 offenbart einen wärmeabgebenden Doppelrohr-Reaktor, etwa für die Synthese von Methanol, umfassend: einen zylinderförmigen Kühlmantel mit einem seitlichen Einlass und einem seitlichen Auslass für ein Kühlmittel, einer in einem oberen Abschnitt des Kühlmantels angeordneten kreisförmigen Platte, die den Innenraum des Kühlmantels in einen unteren Teilbereich zum Durchströmen des Kühlmittels und einen oberen Teilbereich unterteilt, einem an der Platte befestigten Doppelrohr, einem Einlass und einem Auslass für das Reaktionsgas.

Das Doppelrohr besteht aus einem inneren Rohr, einem äußeren Rohr und einem zwischen dem inneren und dem äußeren Rohr ausgebildeten kreisförmigen Raum, in dem ein mit einem Katalysator befüllten Katalysator-Bett ausgebildet ist. Das innere Rohr ist an seinem einen (oberen) Ende als der Einlass, der mit der Außenseite des Reaktors strömungstechnisch in Verbindung steht, ausgebildet und steht an seinem anderen (unteren) Ende mit einem Einlass für das Katalysator-Bett am (unteren) Ende des Doppelrohres in Verbindung. Bei dem anderen (oberen) Ende des kreisförmigen Raums befindet sich ein Auslass des Katalysator-Bettes, der mit dem Auslass für das Reaktionsgas in Verbindung steht. Am unteren Ende des Doppelrohres steht das untere Ende des inneren Rohres mit dem Einlass für das Katalysator-Bett in strömungstechnischer Verbindung. Der Einlass für das Kühlmittel in dem Kühlmantel befindet sich etwa auf der Höhe des Einlasses des Katalysator-Bettes, und der Auslass etwa auf der Höhe des Einlasses des Katalysator-Bettes.

DE 34 42 053 A1 offenbart auch Ausführungsformen eines Doppelrohr-Reaktors mit zwei parallel geschalteten Doppelrohren, einem mit dem Einlass in Verbindung stehenden Erhitzer, zwei flexiblen Schläuchen, die den Erhitzer mit einem jeweiligen inneren Rohr eines Doppelrohres verbinden. In keiner der in DE 34 42 053 A1 offenbarten Ausführungsformen enthält das innere Rohr des Doppelrohres ein Katalysator-Bett.

DE 933 087 offenbart einen Röhrenofen mit gebogenen, zum Umsetzen von gas- oder dampfförmigen Stoffen, insbesondere von Kohlenwasserstoffen und Wasserdampf, mit einer Rohrschlange, die mit einer Katalytmasse gefüllte Rohr-Teilstücke umfasst. Der Röhrenofen umfasst eine ringzylindrische Heizkammer, die umgeben ist von einem Innenmantel, einem aus einem wärmebeständigen und schlecht wärmeleitfähigen Stoff bestehenden Außenmantel und zwischen diesen Mänteln angeordnete elektrische Heizwiderstände, und die mit einem Deckel verschließbar ist. Der Röhrenofen umfasst ferner eine im Inneren der Heizkammer angeordnete Rohrschlange mit vier jeweils senkrecht stehenden Teilstücken, die durch drei Rohrbögen miteinander verbunden sind, und deren Endstücke durch den Deckel hindurch geführt sind.

An den die Rohr-Teilstücke verbindenden Rohrbogen ist ein Einfüllstutzen angesetzt, der durch den Deckel hindurchgeführt ist und durch einen Stopfen verschlossen ist. Durch den Stutzen kann der zur Füllung der Rohr-Teilstücke erforderliche, körnige oder aus kleinen Formkörpern bestehende Katalytmasse in die mittleren Rohrteile sowie in die daran anschließenden Teile der Rohrbögen eingefüllt werden. Die restliche Katalytmasse wird durch die mit Flanschen versehenen Enden der geraden Rohrteile (Endteile) eingebracht. Einer an das Rohr-Teilstück angeschlossenen Leitung wird das Endprodukt entnommen.

Der Röhrenofen der DE 933 087 umfasst eine (einzige) Rohrschlange mit mehreren seriell angeordneten Rohr-Teilstücken. DE 933 087 offenbart nicht, dass ein Reaktor mehrere zueinander parallel geschaltete Reaktorelemente umfasst. DE 933 087 offenbart auch nicht eine gemeinsame Ausgangsstoffzufuhr, die das Parallelschalten mehrerer Reaktorelemente und das parallele Beschicken mit dem Ausgangsstoff mittels der die Gaseintritte gewährleistet.

Bei bisherigen Syntheseanlagen zur Herstellung von kohlenwasserstoff-basierten Energieträgern wie Methanol kommen grosse, komplexe und sehr teuere Synthesereaktoren zum Einsatz. Das entsprechende Know-how zur Auslegung und zur Herstellung dieser Reaktoren und zur Entwicklung geeigneter Katalysatoren liegt bei einigen wenigen Grossfirmen.

Es besteht aber der Bedarf nach kleineren und weniger teueren Synthesereaktoren, zumal der Trend momentan in Richtung Kleinanlagen und sogar in Richtung transportable Anlagen in der Grösse von Seefrachtcontainern geht. Hier sind die großtechnischen Synthesereaktoren nicht geeignet.

Es stellt sich nun die Aufgabe, einen entsprechenden Reaktor und ein entsprechendes Verfahren bereit zu stellen, um ein kohlenstoff- und wasserstoffhaltiges Gas, z.B. H₂- und CO₂-haltiges Synthesegas, effizient und wirtschaftlich sinnvoll zu einem kohlenwasserstoff-basierten Energieträger, wie z.B. Methanol, umsetzen zu können. Ein besonderes Augenmerk gilt dabei der Reduktion der Baugrösse des Synthesereaktors. Ausserdem soll der Synthesereaktor möglichst skalierbar sein, um mit den entsprechenden Bauteilen Reaktoren verschiedenster Kapazität realisieren zu können.

Gemäß Erfindung werden ein Verfahren und ein Reaktor zum Bereitstellenspeicherbarer und transportabler, vorzugsweise methanol-basierter, Energieträger bereit gestellt. Ausserdem wird ein Verfahren zur Verwendung eines solchen Reaktors vorgeschlagen.

Gemäß Erfindung wird ein kohlenstoffhaltiger Gasanteil, vorzugsweise Kohlenstoffoxide, als Kohlenstofflieferant eingesetzt. Der kohlenstoffhaltige Gasanteil wird mit einem Wasserstoffanteil in Anwesenheit eines Katalysators zur Reaktion gebracht, um diese Gase zu einem Energieträger, vorzugsweise einem Alkohol, und besonders vorzugsweise einem methanolhaltigen Produkt umzusetzen.

Vorzugsweise wird Kohlendioxid aus einem Verbrennungsprozess oder einem Oxidationsprozess von Kohlenstoff oder Kohlenwasserstoffen mittels CO₂-Abscheidung entnommen. CO₂ kann zum Beispiel über eine Pipeline oder aber auch in Stahlflaschen oder Tanks bereit gestellt werden. Es kann aber auch Kohlenmonoxid (CO) oder Methangas (CH₄) oder andere fossile oder biogene Gase als kohlenstoffhaltiger Gasanteil eingesetzt werden.

Der Wasserstoff kann über eine Pipeline oder aber auch in Stahlflaschen oder Tanks bereit gestellt werden. Vorzugsweise wird der Wasserstoff jedoch vor Ort mittels Wasserelektrolyse hergestellt. Alternativ kann der Wasserstoff auch durch eine Oxidationsreaktion von elementarem Silizium oder einem anderen elementaren Metall erzeugt werden.

Kohlendioxid als Kohlenstöfflieferant kann gemäß Erfindung auch aus Roherdgas entnommen werden, das je nach Erdgasquelle über 10 % Kohlendioxid-Anteil aufweisen kann. Kohlendioxid kann z.B. auch aus Prozessen des Kalkbrennens oder des Kalzinierens zu Soda entstammen.

Gemäß Erfindung wird ein möglichst konstanter und langzeitiger Anlagenbetrieb einer entsprechenden Silicon-Fire Anlage angestrebt, was durch eine gleichmäßige Beschickung mit einer computergesteuerten Prozesskontrolle erzielt wird.

Die erfindungsgemäße Silicon-Fire Anlage wird so gesteuert und die einzelnen Prozesse werden so miteinander "verknüpft", dass
- der Gesamtertrag und die Qualität (wie z.B. die Reinheit) der Reaktionsprodukte möglichst maximal wird,
- und/oder die CO₂-(Gesamt-)Emission möglichst minimal wird,
- und/oder eine möglichst konstante und langzeitige Anlagenauslastung erzielt wird,
- und/oder die produktspezifischen Investitions- und Betriebskosten der Silicon-Fire Anlage möglichst minimal werden.

Vorzugsweise wird regenerative elektrische Energie zum Betrieb der Silicon-Fire Anlage eingesetzt.

Mit einer Silicon-Fire Anlage wird vorzugsweise Methanol als speicher- und transportierbare Energieform hergestellt. D.h., es werden die erneuerbaren Energien auf chemischem Wege in eine unkritische und relativ einfach speicher- und transportierbare (flüssige) Energieform überführt.

Außerdem kann, je nach Ausführungsform, auch fossiles gasförmiges Methan in einem effizienten chemischen Prozess direkt zu flüssigem Methanol umgesetzt werden, das gegenüber einem Gas wiederum unkritisch und relativ einfach speicher- und transportierbar ist. So lassen sich die aufwendigen Gasverflüssigungsanlagen und die entsprechenden Flüssiggastankschiffe ersetzen.

Die Produktion von flüssigen Kohlenwasserstoffen als relativ einfach speicher- und transportierbare Energieform kann jederzeit heruntergefahren oder gar unterbrochen werden. Die verfahrenstechnischen Anlagenteile zur Herstellung des Kohlenwasserstoffs können relativ einfach und schnell heruntergefahren oder abgeschaltet werden. Hier liegt die Entscheidungshoheit im Verantwortungsbereich des Betreibers der Silicon-Fire Anlage.

Flüssiger Kohlenwasserstoff kann als Energiepuffer dienen. So kann zum Beispiel ein flüssiger Kohlenwasserstoff gespeichert werden, um bei Spitzen-Energiebedarf im elektrischen Verbundnetz zusätzlich elektrische Energie zur Verfügung stellen zu können. Methanol kann bei Bedarf entweder in Wärmekraftwerken verbrannt werden, oder es kann damit in Brennstoffzellen (z.B. Direkt-Methanol Brennstoffzellen; MFC genannt) elektrische Energie erzeugt werden.

Bei Bedarf kann das Methanol vor der Verbrennung katalytisch in ein Spaltgas aus Wasserstoff und Kohlenmonoxid überführt werden. Daraus ergeben sich Vorteile bei gewissen Ümsetzungsprozessen.

Bevorzugte Ausführungsformen der Erfindung basieren auf der Wasserstofferzeugung mit Hilfe elektrischer Energie, die weitmöglich regenerativ erzeugt wird und z.B. aus Wind-, Wasser und/oder Solarkraftwerken stammt. Wasserstoff, der z.B. vor Ort per Elektrolyse oder durch den Einsatz von elementarem Silizium erzeugt wird, braucht also nicht gelagert oder hoch verdichtet oder tief gekühlt verflüssigt und über größere Strecken transportiert zu werden, sondern dient als Zwischenprodukt, das vorzugsweise am Standort seiner Erzeugung der vorgenannten Reaktion zur Erzeugung von Kohlenwasserstoffen zugeführt wird.

Einem energieumwandelnden Prozess, bei dem regenerative Energie in elektrische Energie umgewandelt wird, folgen je nach Ausführungsform der Erfindung z.B. stoffumwandelnde (chemische) Prozesse, nämlich die intermediäre Bereitstellung von Wasserstoff und die Umwandlung des Wasserstoffs zusammen mit einem Kohlenstoffträger (z.B. Kohlendioxid) zu Methanol.

Methanol kann aber gemäss Erfindung auch unter Einsatz fossiler Energie öder durch einen intelligenten Energiemix (siehe z.B. WO2010069622A1) aus fossiler und regenerativer Energie erzeugt werden.

Unter Beachtung entsprechender energietechnischer, anlagentechnischer und wirtschaftlicher Vorgaben, zusammen mit der Forderung nach schonender Nutzung aller stofflichen, energetischen und ökonomischen Ressourcen, wird gemäß Erfindung eine neue energietechnische Lösung bereitgestellt.

Weitere vorteilhafte Ausführungsformen sind der Beschreibung, den Figuren und den abhängigen Ansprüchen zu entnehmen.

In den Zeichnungen sind verschiedene Aspekte der Erfindung schematisch dargestellt.
- Fig. 1:: zeigt ein Schema, welches das Prinzip der Klimaneutralität durch die Investition in, öder das Betreiben von Ausgleichsprojekten darstellt;
- Fig. 2:: zeigt ein Schema, das die grundlegenden Schritte des Verfahrens, gemäß einer der eingangs erwähnten internationalen Patentanmeldungen, respektive einer entsprechenden Silicon-Fire Anlage wiedergibt;
- Fig. 3:: zeigt ein Schema, das die grundlegenden Schritte des Verfahrens gemäß Erfindung, respektive einer entsprechenden Silicon-Fire Anlage wiedergibt;
- Fig. 4A:: zeigt eine schematische Seitenansicht, die grundlegende Aspekte eines Reaktorelements als Reaktionsrohr, gemäß Erfindung, erkennen lässt;
- Fig. 4B:: zeigt eine schematische Ausschnittsvergrösserung im Teilschnitt, die Details des Reaktorelements nach Fig. 4A erkennen lässt;
- Fig. 5A:: zeigt ein Seitenansicht eines weiteren Reaktorelements gemäß Erfindung;
- Fig. 5B:: zeigt eine Draufsicht des Reaktorelements nach Fig. 5A;
- Fig. 5C:: zeigt eine Seitenansicht des oberen Bereichs des Reaktorelements nach Fig. 5A in einer Detailansicht X;
- Fig. 5D:: zeigt einen Schnitt durch den oberen Bereich des Reaktorelements nach Fig. 5A;
- Fig. 5E:: zeigt einen Schnitt entlang der Schnittlinie C-C in Fig. 5D;
- Fig. 6A:: zeigt eine Seitenansicht eines weiteren Reaktorelements gemäß Erfindung;
- Fig. 6B:: zeigt eine Draufsicht des Reaktorelements nach Fig. 6A und die mögliche gewinkelte Variante der Anordnung der einzelnen Teile des Reaktionsrohres nach Fig. 5B;
- Fig. 7A:: zeigt eine seitliche Schnittansicht (entlang der Schnittlinie A-A in Fig. 7B) eines Reaktors gemäß Erfindung;
- Fig. 7B:: zeigt eine Draufsicht des Reaktors nach Fig. 7A;
- Fig. 7C:: zeigt eine seitliche Außenansicht des Reaktors nach Fig. 7A;
- Fig. 8:: zeigt eine Draufsicht eines Reaktors nach Fig. 7A, wobei die beiden oberen Ringleitungen samt Zufuhrleitungen in schematischer Form angedeutet sind;

Der Begriff Energieträger wird hier verwendet für flüssige Stoffe, die entweder direkt als Kraftstoff oder Brennstoff eingesetzt werden können. Hier geht es insbesondere um Methanol 108, bzw. um methanolhaltige Energieträger. Der Begriff "methanolhaltiges Produkt" wird hier verwendet, da das Produkt, das am Ausgang eines Reaktors bereit gestellt wird, nicht zu hundert Prozent aus Methanol besteht. Es handelt sich vielmehr um ein sogenanntes physikalisches Gemisch aus Methanol und Wasser, das hier als methanolhaltiges Produkt bezeichnet wird. Durch einen anschließenden Destillationsprozess kann dann reines Methanol gewonnen werden.

Als Prozessrichtung wird hier die Richtung von der Eingangsseite zur Ausgangsseite, d.h. die Flussrichtung im Inneren eines Reaktors 10 bezeichnet.

Im Falle von Methanol 108 als Energieträger sollten gewisse Rahmenbedingungen bei Herstellung, Lagerung und beim Transport eingehalten werden, die ähnlich sind wie die Bedingungen zur Handhabung von anderen fossilen flüssigen Kraft- und Brennstoffen. Hier kann problemlos die existierende Infrastruktur genutzt werden. Auf der Materialseite können gewisse Anpassungen erforderlich sein, um z.B. den korrosiven Eigenschaften des Methanols Rechnung zu tragen. Auch die Sicherheitsmaßnahmen z.B. hinsichtlich Gesuridheits-, Brand- und Explosionsschutz sind anzupassen.

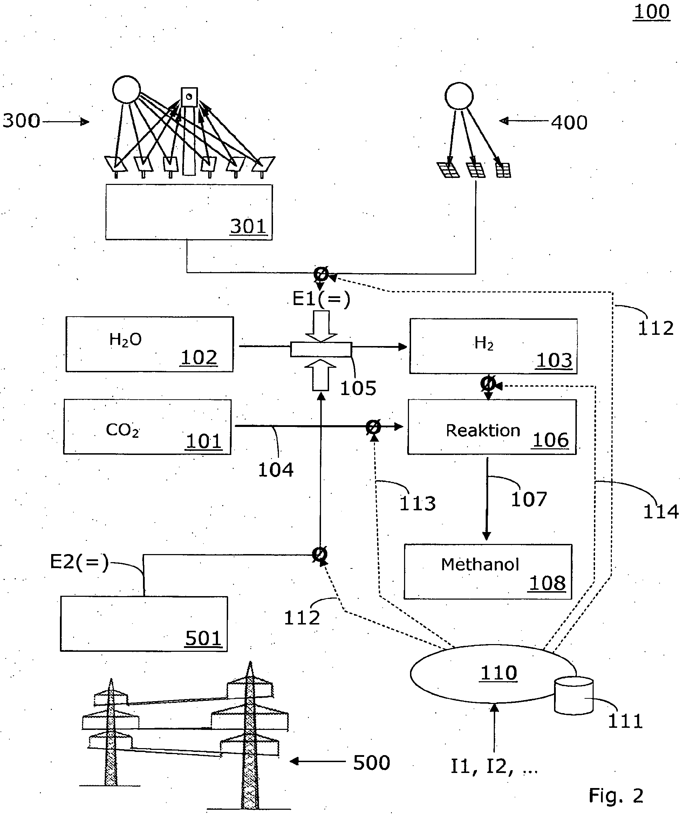

Fig. 2 zeigt in einer schematischen Blockdarstellung die wichtigsten Bausteine/Komponenten, respektive Verfahrensschritte, einer Silicon-Fire Anlage 100 gemäß einer der eingangs erwähnten internationalen Patenanmeldungen. Diese Anlage 100 ist so ausgelegt, dass ein Verfahren zum Bereitstellen speicherbarer und transportabler Energieträger 108 ausgeführt werden kann. Das entsprechende Verfahren basiert auf den folgenden grundlegenden Schritten.

Es wird z.B. Kohlenstoffdioxid 101 als Kohleristofflieferant bereitgestellt. Die erforderliche elektrische Gleichstromenergie E1 wird hier weitmöglichst mittels erneuerbarer Energietechnik erzeugt und der Silicon-Fire Anlage 100 zur Verfügung gestellt. Besonders als erneuerbare Energietechnik geeignet sind Solarthermieanlagen 300 und Photovoltaikanlagen 400, die auf Solarmodulen basieren. Es kann z.B. auch Wasserkraft eingesetzt werden. Es ist auch möglich, eine Kombination von mehreren Anlagentypen 300 und 400 vorzusehen, da der Flächenbedarf, bezogen auf die elektrische Leistung, einer Solarthermieanlage 300 kleiner ist als der einer Photovoltaikanlage 400.

Es wird gemäß Fig. 2 eine Wasserelektrolyse 105 unter Einsatz der elektrischen Gleichstromenergie E1 durchgeführt, um Wasserstoff 103, oder Wasserstoffionen, als Zwischenprodukt zu erzeugen.

In Fig. 2 ist eine Anlage 100 dargestellt, die so aufgebaut ist, dass sie die eingangs erwähnten Nachteile vermindert oder ausgleicht. Aus diesem Grund wird bei der Silicon-Fire Anlage 100 vorzugsweise eine wirtschaftlich und ökologisch optimale Kombination regenerativer Stromversorgung (durch die Anlagen 300 und/oder 400) und konventioneller Stromversorgung, hierdurch einen Teil eines Verbundnetzes 500 dargestellt, realisiert. Diese Silicon-Fire Anlage 100 sieht daher vor, die regenerative elektrische Energie E1 weitgehend direkt entsprechend ihrem Anfall für chemische Reaktionen (hier die Elektrolysereaktion 105) zu nutzen und damit chemisch zu binden und zu speichern. Ein weiterer Anteil der benötigten Energie wird hier beispielsweise aus dem Verbundnetz 500 bezogen. Dieser Anteil wird in Gleichstrom(energie) E2 umgewandelt. Zu diesem Zweck kommt ein entsprechender Umwandler 501 zum Einsatz, wie in Fig. 2 in schematischer Form angedeutet. Die entsprechenden Anlagenteile oder-komponenten werden hier auch als Energieversorgungsanlage 501 bezeichnet.

Mittels einer intelligenten Anlagensteuerung 110 wird die Energieversorgung der Anlage 100 nach Fig. 2 gesteuert und geregelt. Es wird im Prinzip der jeweils momentan verfügbare überschüssige Energieanteil E2 aus dem Verbundnetz 500 bezogen, während der andere Energieanteil (hier E1) soweit möglich aus einem Anlage-bezogenen Solarkraftwerk 300 und/oder 400 (und/oder aus einem Windkraftwerk und/oder aus einem Wasserkraftwerk) bezogen wird. Hier kommt es also zu einer intelligenten Umkehrung des bisherigen Prinzips, bei dem die Energieschwankungen erneuerbarer Energieanlagen 300; 400 durch Zu- und Abschalten konventioneller Kraftwerke abgefangen werden. Man braucht daher zum Betreiben einer Silicon-Fire Anlage 100 keine zusätzlichen Leistungs- und Frequenzregelkapazitäten für die regenerativen Kraftwerksanlagen im Verbundnetz 500 vorzuhalten. Dieses Prinzip ermöglicht es dem Betreiber einer Silicon-Fire Anlage 100, zusätzliche technische und wirtschaftliche Parameter bei der Steuerung der Anlage 100 einzubeziehen. Bei diesen Parametern handelt es sich um sogenannte Input-Größen I1, I2, usw., die von der Steuerung 110 in Entscheidungen eingezogen werden. Ein Teil der Parameter kann innerhalb der Steuerung 110 in einem Parameterspeicher 111 vorgegeben werden. Ein anderer Teil der Parameter kann von außen kommen. Hier können zum Beispiel Preis- und/oder Verfügbarkeitsinformationen vom Betreiber des Verbundnetzes 500 eingehen.

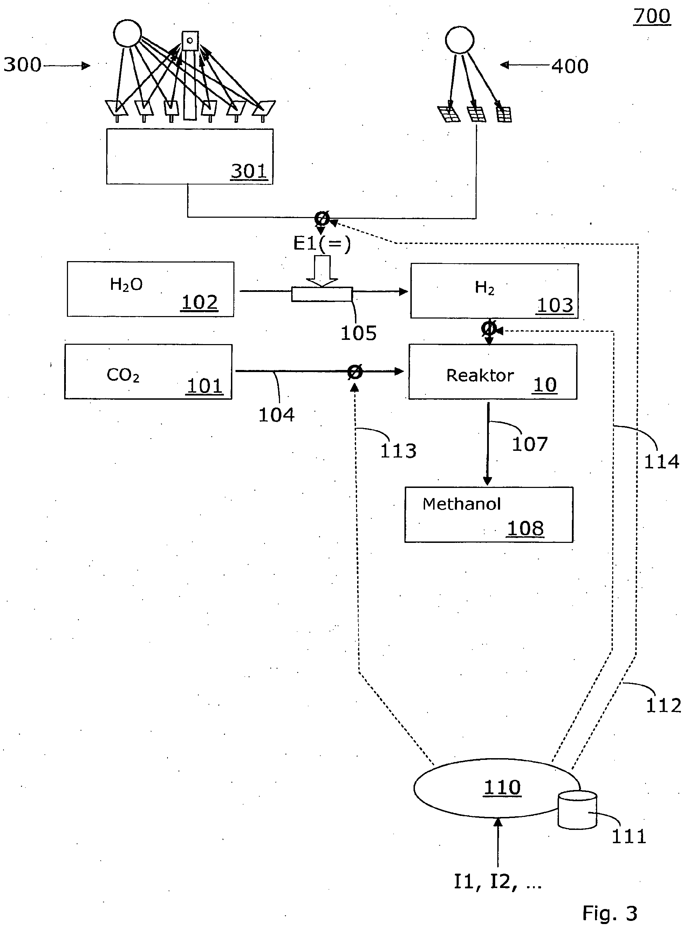

In Fig. 3 ist nun eine erfindungsgemäße Anlage 700 schematisch dargestellt, die so aufgebaut ist, dass sie die eingangs erwähnten Nachteile vermindert oder ausgleicht. Ein Teil dieser Anlage 700 entspricht der Anlage 100 nach Fig. 2. In dieser Beziehung wird daher auf die vorausgehende Beschreibung der entsprechenden Elemente verwiesen.

Es wird bei dieser Ausführungsform, wie beschrieben, durch eine Wasserelektrolyse 105 hochreiner Wasserstoff 103 erzeugt, der hier z.B. zu Methanol 108 umgesetzt wird. Die Energie hierzu stammt bei dieser Ausführungsform ganz oder weitestgehend (vorzugsweise zu mehr als 80%) aus regenerativen Energiequellen 300 und/oder 400 (oder aus anderen regenerativen Energiequellen).

Es können eine Reihe von Steuer- oder Signalleitung vorgesehen sein, wie anhand der beispielhaft gezeigten Leitungen 112, 113 und 114 dargestellt. Diese Leitungen 112, 113 und 114 steuern Energie- oder Masseströme der Anlage 700.

In der Anlagensteuerung 110 sind sogenannte software-basierte Entscheidungsprozesse implementiert. Ein Prozessor der Steuerung 110 führt eine Steuerungssoftware aus und fällt unter Berücksichtigung von Parametern programmierte Entscheidungen. Diese Entscheidungen werden in Schalt- oder Steuerungsbefehle umgesetzt, die zum Beispiel über Steuer- oder Signalleitungen 112, 113, 114 die Steuerung/Regelung von Energie- und Massenströmen bewirken.

Gemäß einer besonders bevorzugten Ausführungsform der Erfindung wird Kohlendioxid 101 als gasförmiger Kohlenstofflieferant eingesetzt, wie in Fig. 3 schematisch angedeutet. Vorzugsweise wird das Kohlendioxid 101 aus einem Verbrennungsprozess oder einem Oxidationsprozess über CO₂-Abscheidung (z.B. eine Silicon-Fire Rauchgasreinigungsanlage) entnommen. Das Kohlendioxid 101 kann aber auch von Röherdgas abgetrennt und bereitgestellt werden. Das Kohlendioxid 101 kann auch aus anderen Quellen kommen. Vorzugsweise wird das Kohlendioxid 101 über eine Pipeline, eine Stahlflasche oder einen Tank bereit gestellt.

Weiterhin wird bei der gezeigten Ausführungsform elektrische Gleichstromenergie E1 bereitgestellt. Die Gleichstromenergie E1 wird vorzugsweise weitgehend regenerativ (z.B. durch eine der Anlagen 300 und/oder 400 in Fig. 3) erzeugt. Die Gleichstromenergie E1 wird bei der gezeigten Ausführungsform zum Durchführen einer Wasserelektrolyse eingesetzt, um Wasserstoff 103 als Zwischenprodukt zu erzeugen. Die Elektrolyseanlage, respektive das Durchführen einer solchen Elektrolyse, ist in Fig. 3 durch das Bezugszeichen 105 gekennzeichnet. Das Kohlendioxid 101 wird mit dem Wasserstoff 103 zusammen geführt. Das entsprechende Gas wird hier als Ausgangsstoff AS bezeichnet. Der Ausgangsstoff AS wird zur Reaktion (Methanolsynthese in einem Reaktor 10, gemäss Erfindung) gebracht, um die gasförmigen (Zwischen-)Produkte 101, 103 z.B. zu Methanol 108 umzusetzen. Die Reaktion wird in dem erfindungsgemässen Reaktor 10 durchgeführt. Die Entnahme, respektive das Bereitstellen des Methanols 108, ist in Fig. 3 durch das Bezugszeichen 107 gekennzeichnet.

Im Folgenden werden weitere grundlegende Details dieses Verfahrens und der entsprechenden Silicon-Fire Anlage 700 beschrieben.

Um Wasserstoff 103 als Zwischenprodukt erzeugen zu können, eignet sich eine Wasser-Elektrolyse unter Einsatz von Gleichstrom E1. Der benötigte Wasserstoff 103 wird in einer Elektrolyseanlage 105 durch die Elektrolyse von Wasser H₂O nach folgender Gleichung hergestellt:

H₂O-286,02 kJ = H₂ + 0,5 O₂. (Reaktion 1)

Die benötigte (elektrische) Energie E1 für diese Reaktion von 286,02 kJ/mol entspricht 143010 kJ pro kg H₂.

Die Synthese des Methanols 108 (CH₃OH) kann in dem Reaktor 10 der Silicon-Fire Anlage 700 nach der exothermen Reaktion zwischen Kohlendioxid 101 (CO₂) und Wasserstoff 103 (H₂) wie folgt erfolgen:

CO₂ + 3 H₂ = CH₃OH + H₂O - 49,6 kJ (Methanol-Wassergemisch, dampfförmig) (Reaktion 2)

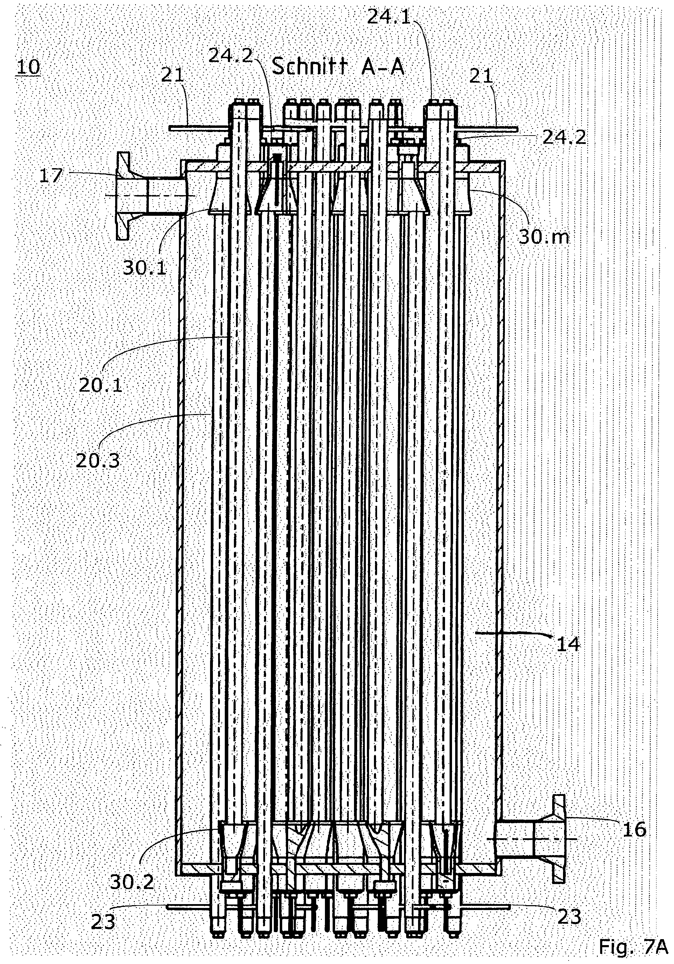

Die entstehende Reaktionswärme von 49,6 kJ/mol = 1550 kJ pro kg Methanol = 0,43 kWh pro kg Methanol 108 wird aus dem entsprechenden Reaktor 10 abgeführt. Zu diesem Zweck umfasst der Reaktor 10 einen Fluidraum 14 (siehe z.B. Fig. 7A), d.h. die Reaktorröhren 20.n sind im Reaktor 10 von einem Reaktormantel umgeben und durch ein Fluid (vorzugsweise Wasser) gekühlt.

Typische Synthesebedingungen im Synthesereaktor 10 sind ca. 50 bis 80 bar und ca. 270 °C. Die Reaktionswärme kann z.B. an andere Anlagenelemente, wie zum Beispiel einem Verdampfer der Destillationskolonne oder an andere nachgeschaltete Anlagenbereiche "übergeben" werden.

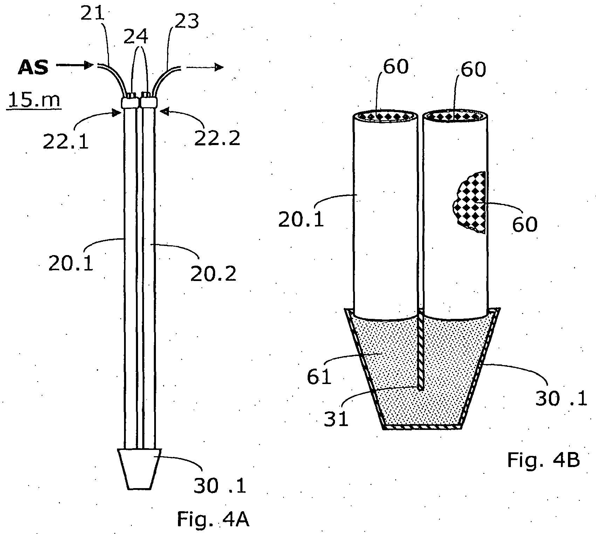

Die (Methanol-)Synthese wird gemäss Erfindung unter Einsatz eines Katalysators 60 (siehe z.B. Fig. 4B) durchgeführt, um Reaktionstemperatur, Reaktionsdruck sowie Reaktionsdauer im Vergleich zu anderen Verfahren niedrig zu halten und um sicher zu stellen, dass als Reaktionsprodukt hochreiner flüssiger Kohlenwasserstoff (z.B. Methanol 108) entsteht.

Falls sich die Silicon-Fire Anlage 700 in der Nähe einer CO₂-Quelle befindet, kann auf eine Verflüssigung von CO₂ für den Transport verzichtet werden. Ansonsten ist es nach dem Stand der Technik relativ einfach, das CO₂ zu verflüssigen und auch über große Entfernungen zu einer Silicon-Fire Anlage 700 zu bringen. Bei einem Verzicht auf Verflüssigung und ggf. auf Speicherung und Transport über längere Entfernung ist das CO₂ unter Berücksichtigung von CO₂-Vermeidungs-Gutschriften voraussichtlich kostenneutral verfügbar. Auch im Falle eines Transports sind die Kosten für das "Erwerben" des CO₂ relativ gering.

In Fig. 3 ist anhand des gestrichelten Pfeils 112 angedeutet, der von der Steuerung 110 ausgeht, dass die Steuerung 110 den Energiestrom E1 regelt. Der Pfeil 112 stellt eine Steuer- oder Signalleitung dar. Es sind auch andere mögliche Steuer- oder Signalleitungen 113, 114 dargestellt. Die Steuer- oder Signalleitung 113 regelt zum Beispiel die CO₂-Menge, die für die Reaktion 106 zur Verfügung steht. Wenn zum Beispiel weniger Wasserstoff 103 produziert wird, dann muss auch proportional weniger CO₂ zugeführt werden. Die optionale Steuer- oder Signalleitung 114 kann zum Beispiel die H₂-Menge regeln. Eine solche Regelung ist dann sinnvoll, wenn es einen Wasserstoff-Pufferspeicher gibt, dem man Wasserstoff 103 entnehmen kann, auch wenn im Moment kein Wasserstoff oder weniger Wasserstoff durch Elektrolyse 105 (oder durch den Einsatz elementaren Siliziums) produziert wird.

Untersuchungen haben ergeben, dass es besonders wirtschaftlich und umwelttechnisch sinnvoll ist, wenn die Silicon-Fire Anlage 100 so ausgelegt oder gesteuert wird, dass zwischen 15% und 40% des Methanols 108 aus regenerativer Energie erzeugt wird, während weiteres Methanol für die Ergänzung zu 100% aus anderen Kohlenwasserstoffen (z.B. aus Methangas) bereitgestellt wird.

Besonders bevorzugt ist eine Ausführungsform des Betriebskonzepts der Anlage 100, die den Bezug kostengünstiger elektrischer Energie in den Schwachlastzeiten aus einem Verbundnetz 500 vorsieht (wie in Fig. 2).

Details einer besonders bevorzugten Ausführungsform eines Reaktors 10 zur Synthese von Ethanol 108 ist in den Figuren 4A bis 7C gezeigt. Die Ausführungen, die im Folgenden zur Synthese von Methanol 108 gemacht werden, lassen sich auch auf die Synthese anderer flüssiger Kohlenwasserstoffe übertragen.

Das Methanol 108 wird, wie bereits beschrieben, unter Einsatz eines Ausgangsstoffes AS, der z.B. CO₂-Gas 101 und Wasserstoffgas 103 enthält, synthetisiert. Der entsprechende Reaktor 10 umfasst ein Reaktorelement 15.1 oder mehrere parallel zueinander angeordnete Reäktorelemente 15.m (m ist hier eine ganze Zahl, die grösser gleich 2 ist). Ein Reaktorelement 15.m umfasst bei allen Ausführungsformen mindestens n=2 Reaktorröhren 20.n (n ist hier eine ganze Zahl, die grösser gleich 2 ist). Ein Reaktorelement 15.m mit n=2 Reaktorröhren 20.1 und 20.2 ist in den Figuren 4A und 4B gezeigt. Bei einer solchen Anordnung, die bei n=2 Reaktorröhren 20.1 und 20.2 stets ein Umlenkelement 30.1 (hier ein 180-Grad-Umlenkement) umfasst, ergibt sich eine Konstellation, bei der sowohl der Gaseintritt 21 für den Ausgangsstoff AS als auch der Produktauslass 23 entweder am oberen Ende des Reaktorelements 15.m (wie in Fig. 4A gezeigt) oder am unteren Endes des Reaktorelements 15.m sitzen. Dadurch ergeben sich bestimmte konstruktive Bedingungen, wenn es um einen Reaktor 10 geht, der eine grössere Anzahl (z.B. m grösser 6) von Reaktorelementen 15.m in einem Bündel umfasst.

Der Begriff "Parallelanordnung" wird hier verwendet, um zu beschreiben, dass sie einzelnen Reaktorelemente 15.m aus reaktionstechnischer und strömungstechnischer Sicht parallel in Volumen. und Zeit zueinander vom Ausgangsstoff AS durchlaufen werden. Da der Ausgangsstoff AS beim Durchlaufen der Reaktorröhren 20.n der Reaktorelemente 15.m sukzessive zu einem methanolhaltigen Stoff umgesetzt wird, wird die Fluidzwischenstufe, die im Reaktor 10 entsteht, hier als Reaktionsfluid bezeichnet. Auf der Eingangsseite des Reaktors 10 liegt die Methanolkonzentration des Reaktionsfluids bei Null und die Konzentration des gasförmigen Ausgangsstoffes AS bei ca. 100%. In Richtung Ausgangsseite verschieben sich die entsprechenden Konzentrationen gegenläufig bis am Ausgang (am Produktauslass 23) ein methanolhaltiger Stoff mit einer hohen Methanolkonzentration (vorzugsweise ein Methanol-Wassergemisch im Verhältnis 1:2) gebildet ist.

Es ist ein Vorteil der Erfindung, dass jedes Reaktorelement 15.m als ein eigener singulärer Synthesereaktor ausgebildet ist. Durch die parallele Anordnung von m Reaktorelementen 15.1 bis 15.m lässt sich die Kapazität des Reaktors 10 (entsprechend der Zahl m) variieren. Die Zahl n hingegen hat einen direkten Einfluss auf die Baugrösse des Reaktors 10.

Jedes der Reaktorelemente 15.m weist einen Gaseintritt 21 an einem ersten Röhrenende 22.1 und einen Produktauslass 23 an dem zweiten Röhrenende 22.2 auf. Jedes der Reaktorelemente 15.m hat mindestens ein Umlenkelement 30.k (vorzugsweise in Form eines 180-Grad-Umlenkelements), das zwischen dem ersten Röhrenende 22.1 und dem zweiten Röhrenende 22.2 des jeweiligen Reaktorelements 15.m liegt.

Wenn ein Reaktorelement 15.m n=2 Reaktorröhren 20.1 und 20.2 umfasst, dann weist sie k=1 180-Grad-Umlenkelemente 30.k auf. Wenn ein Reaktorelement 15.m n=3 Reaktorröhren 20.1, 20.2 und 20.3 umfasst, dann weist sie k=2 180-Grad-Umlenkelemente 30.k auf. Dieses Prinzip lässt sich entsprechend auch auf Reaktorelemente 15.m mit mehr als drei (d.h. n grösser 3) Reaktorröhren 20.n übertragen.

Gemäss Erfindung sind die Reaktorröhren 20.n eines Reaktorelements 15.m gegenläufig angeordnet. Als gegenläufige Anordnung wird eine räumliche, strömungstechnische Konstellation bezeichnet, bei der das Reaktionsfluid z.B. in einer Reaktorröhre 20.1 nach unten abwärts strömt, während das Reaktionsfluid dann nach der Umlenkung in einem Umlenkelement 30.1 in einer zweiten Reaktionsröhre 20.1 nach oben strömt. Vorzugsweise sitzen die Reaktorröhren 20.n eines Reaktorelements 15.m bei allen Ausführungsformen dicht aneinander. Vorzugsweise beträgt der gegenseitige lichte Abstand (Aussenwand zu Aussenwand) weniger als der Aussendurchmesser eines der Reaktorrohre 20.n.

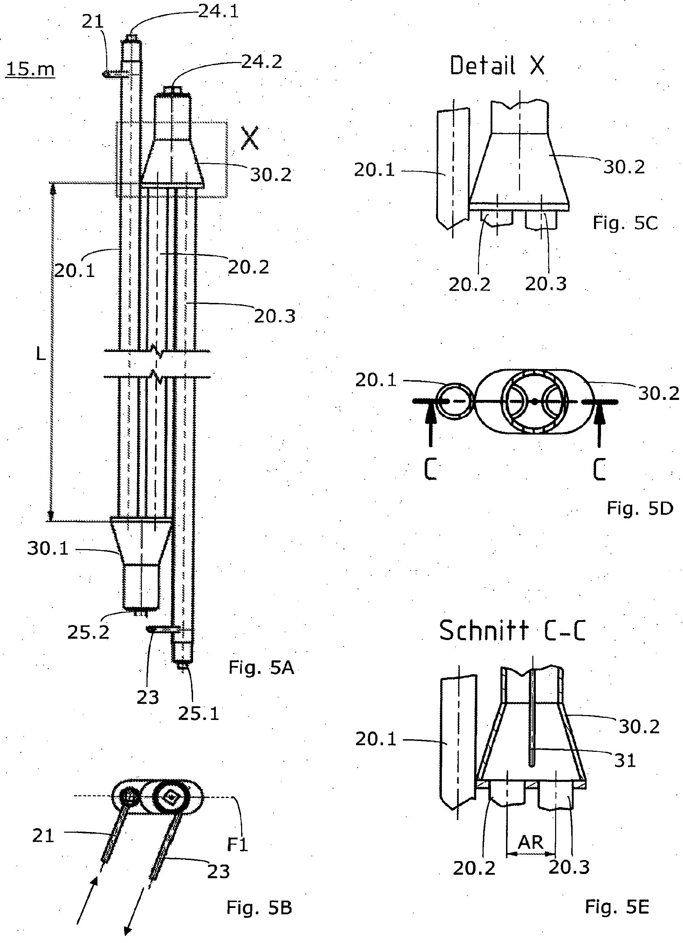

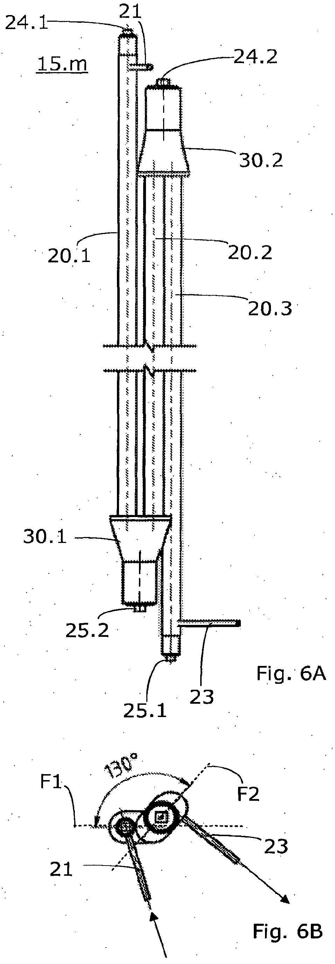

In den Figuren 5A - 5E, 6A, 6B sind Reaktorelemente 15.m mit n=3 Reaktorröhren 20.1, 20.2 und 20.3 und k=2 180-Grad-Umlenkelementen 30.1, 30.2 gezeigt. Der Vorteil einer ungeraden Anzahl n liegt darin, dass die Einlassseite und die Auslassseite an gegenüberliegenden Enden des Reaktorelements 15.m liegen. Dadurch gewinnt man Platz für das Anordnen eines gemeinsamen Eingangssammlers oder -verteilers 11.1, 11.2 (z.B. in Form einer Ringleitung) auf der einen Seite und für das Anordnen des/der Produktauslässe 23 auf der anderen Seite.

Jedes der Reaktorelemente 15.m umfasst mindestens eine obenliegende Befüllöffnung 24.1 zum Einfüllen eines Katalysators 60 für die Synthese von Methanol. Vorzugsweise umfasst bei allen Ausführungsformen jede Reaktorröhre 20.n und/oder jedes obenliegende Umlenkelement 30.2 eine eigene Befüllöffnung 24.1, bzw. 24.2. Die Befüllöffnungen 24.2 an den obenliegenden Umlenkelementen 30.2 sind vorzugsweise bei allen Ausführungsformen etwas grösser als die obenliegenden Befüllöffnungen 24.1, da durch das Umlenkelement 30.2 zwei Reaktorröhren 20.2, 20.3 von oben befüllt werden. Es können, je nach Bedarf, auch untenliegende Entleerungsöffnungen 25.1, 25.2 vorgesehen sein (siehe z.B. Fig. 5A und Fig. 6A). Die Entleerungsöffnungen 25.2 an den untenliegenden Umlenkelementen 30.1 sind vorzugsweise bei allen Ausführungsformen etwas grösser als die untenliegenden Entleerungsöffnungen 25.1, da durch das Umlenkelement 30.1 zwei Reaktorröhren 20.1, 20.2 von unten entleert werden.

Durch die Befüllöffnungen 24.2 kann der Katalysator 60 und/oder inertes Material 61 eingefüllt werden. Durch die Entleerungsöffnungen 25.2 kann der Katalysator 60 und/oder das inerte Material 61 entleert werden. Die Befüllöffnungen 24.1, 24.2 und/oder Entleerungsöffnungen 25.1, 25.2 können bei allen Ausführungsformen der Erfindung vorgesehen sein.

Die Befüllöffnungen 24.1, 24.2 und/oder die Entleerungsöffnungen 25.1, 25.2 sind vorzugsweise bei allen Ausführungsformen z.B. mit einem Schraubverschluss oder mit einer Kappe verschliessbar.

Das Parallelanordnen der Reaktorröhren 20.n wird dadurch gewährleistet, dass die Gaseintritte 21 mehrerer Reaktorelemente 15.m von einer gemeinsamen Sammelleitung für das Synthesegas 11.1, 11.2 (vorzugsweise in Form einer gemeinsamen Ringleitung) mit dem Ausgangsstoff AS beschickbar sind, und dass die Produktauslässe 23 mehrerer Reaktorelemente 15.m strömungstechnisch mit einem nachgeschalteten Anlagenbereich (z.B. einer Destillationskolonne) verbindbar sind.

Im Folgenden sind weitere Details eines Reaktorelements 15.m nach Fig. 5A - 5E beschrieben. In diesen Figuren ist ein Reaktorelement 15.m gezeigt, das speziell für die Anordnung in einem Aussenring (Aussenbündel) eines Reaktors 10 aufgelegt ist. Das Reaktorelement 15.m umfasst n=3 Reaktorröhren 20.1, 20.2, 20.3. Es hat eine erste obenliegende Befüllöffnung 24.1 und einen oben liegenden Gaseintritt 21. Die erste Reaktorröhre 20.1 verläuft senkrecht nach unten und mündet in einem ersten Umlenkelement 30.1. An dem ersten Umlenkelement 30.1 ist eine untenliegende Entleerungsöffnung 25.2 vorgesehen. Von dem ersten Umlenkelement 30.1 erstreckt sich eine zweite Reaktorröhre 20.2 senkrecht nach oben und mündet in einem zweiten Umlenkelement 30.2. An dem zweiten Umlenkelement 30.2 ist eine obenliegende Befüllöffnung 24.2 vorgesehen. Von dem zweiten Umlenkelement 30.2 erstreckt sich eine dritte Reaktorröhre 20.3 senkrecht nach unten. Die dritte Reaktorröhre 20.3 hat eine untenliegende Entleerungsöffnung 25.1 und einen unten liegenden Produktauslass 23.

Die gezeigte Konstellation hat eine effektive Reaktionslänge LE (Reaktorstrecke genannt), die 3 mal der Einzellänge L entspricht, da die drei Reaktorröhren 20.1, 20.2, 20.3 einen gefalteten (mäanderförmigen) Verlauf für das Reaktionsfluid bieten.

In Fig. 5C ist zu erkennen, dass das Umlenkelement 30.2 eine Trichterform haben kann. Diese Form ist bevorzugt. Wichtig ist jedoch nicht die Aussenhülle, sondern der Innenraum, der so ausgelegt sein muss, dass es eine strömungstechnische Verbindung von einer Reaktorröhre 20.2 zu der nächsten Reaktorröhre 20.3 gibt, die keine störende Querschnittsreduktion oder Barriere bildet. In Fig. 5D kann man von oben her in das Innere des Umlenkelements 30.2 blicken. In Fig. 5E ist ein Schnitt gezeigt. In der Schnittdarstellung ist zu erkennen, dass der Innenraum hier trichterförmig ist.

Vorzugsweise kann jedes Umlenkelement 30.k im Innenraum eine Trennwand 31 aufweisen, die zum getrennten Befüllen der einzelnen Reaktorröhren 20.n dient. Diese Trennwand 31 kommt vorzugsweise nur im Moment des Befüllens zum Einsatz. Es ist auch möglich beim Befüllen eine der beiden Reaktorröhren im Inneren des Umlenkelements 30.k temporär abzudecken, damit in diesem Moment die andere Reaktorröhre befüllt werden kann.

In Fig. 5B ist zu erkennen, dass der Gaseinlass 21 und der Produktauslass 23 z.B. schräg angeordnet sein können. Die Richtungen werden vorzugsweise so gewählt, dass sich eingangsseitig eine gemeinsame Ausgangsstoffzufuhr 11 als Sammler/Verteiler anbringen lässt. Ausgangsseitig werden die Produktauslässe 23 vorzugsweise so angeordnet, dass sich zwei oder mehr derselben zusammenfassen lassen.

Die Reaktorelemente 15.m sind in folgender Hinsicht besonders. Sie werden so befüllt oder mit dem Katalysator 60 versehen, dass sich eine Reaktorstrecke ergibt, die einen Katalysatorabschnitt umfasst, der von einem Abschnitt mit inertem Material 61 gefolgt wird. Nach dem Abschnitt mit dem inerten Material 61 folgt erneut ein Katalysatorabschnitt. In Fig. 4B ist dieses Prinzip zu erkennen. Die erste Reaktorröhre 20.1 ist mit dem Katalysator 60 befüllt. Das Umlenkelement 30.1 ist mit dem inerten Material 61 gefüllt und die zweite Reaktorröhre 20.2 ist wiederum mit dem Katalysator 60 befüllt. In beiden Reaktorröhren 20.1, 20.2 ergeben sich Katalysatorsäulen oder -abschnitte, die sich auf dem inerten Material 61 abstützen. Dadurch, dass das inerte Material 61 in das Umlenkelement 30.1 eingebracht wurde, kann kein Katalysator 60 in den Bodenbereich (untersten Bereich) des Umlenkelements 30.1 eindringen.

Der Katalysator 60 wird in den Reaktorröhren 20.n typischerweise einem Reduktionsschritt unterzogen, wobei der Katalysator 60 eine Volumenreduktion erfährt, die mindestens teilweise durch Nachrutschen, Nachfüllen oder Nachgleiten des inerten Materials 61 kompensiert wird. Das inerte Material 61 kann auch nachrücken oder Hohlräume ausfüllen, die sich beim Reduzieren des Katalysators ergeben. Ausserdem verhindert das inerte Material 61 strömungsbedingtes Hochspringen des Katalysators 60 in aufsteigenden Reaktorröhren (z.B. in Reaktorröhre 20.2).

Das inerte Material 61 kommt bei allen Ausführungsformen zum Einsatz.

In Fig. 5B ist zu erkennen, dass die drei Reaktorröhren 20.1, 20.2, 20.3 in einer gemeinsamen ersten Ebene F1 liegen, bzw. stehen. In Fig. 6B ist zu erkennen, dass die drei Reaktorröhren 20.1, 20.2, 20.3 auch eine Winkelanordnung haben können, bei der eine erste äussere Reaktorröhre 20.1 mit der mittleren Reaktorröhre 20.2 in einer gemeinsamen ersten Ebene F1 und eine zweite äussere Reaktorröhre 20.3 mit der mittleren Reaktorröhre 20.2 in einer gemeinsamen zweiten Ebene F2 liegen, bzw. stehen. Dadurch lassen sich bei allen Ausführungsformen sehr dichte Packungen bzw. Bündelungen erreichen.

Die Beschreibung der Figuren 4A bis 4E lässt sich 1:1 auf die Figuren 5A, 5B übertragen. Die Ausführungsform in den Figuren 5A, 5B unterscheidet sich im Wesentlichen nur durch die Winkelanordnung. Die Winkelanordnung (hier mit einem Winkel von 130 Grad) wurde gewählt, um die entsprechenden Reaktorelemente 15.m im inneren Bündel eines Reaktors 10 anordnen zu können. Der Gaseinlass 21 und der Produktauslass 23 weisen hier vorzugsweise in unterschiedliche Richtungen.

In den Figuren 7A bis 7C ist ein Reaktor 10 gezeigt, der gemäss Aufgabenstellung entwickelt und optimiert wurde. Der Reaktor 10 umfasst hier ein Bündel von Reaktorelementen 15.m. Im konkret gezeigten Beispiel umfasst der Reaktor 10 ein innenliegendes Bündel mit 8 Reaktorelementen 15.m gemäss Fig. 6A, 6B und aussenliegendes Bündel mit 12 Reaktorelementen 15.m gemäss Fig. 5A - 5E (d.h. m=20). Um die Darstellung in den 7A bis 7C nicht durch eine übermäßige Anzahl an Bezugslinien und Referenzzeichen zu stören, wurde nur ein Teil der Elemente mit Bezugslinien und Referenzzeichen versehen. Der Reaktor 10 umfasst vorzugsweise einen Fluidraum 14, der hier einen Zylinderform hat. Der Fluidraum 14 umgibt das gesamte Bündel der Reaktorelemente 15.m, wobei lediglich die oberen und unteren Enden der Reaktorelemente aus dem Fluidraum 14 heraus ragen. Somit liegen die ersten und zweiten Befüllöffnungen 24.1, 24.2 sowie die eingangsseitigen Gaseintritte 21 und die ausgangsseitigen Produktauslässe 23 außerhalb des Fluidraumes 14. Der Fluidraum dient dazu in einer bevorzugten Betriebsweise des Reaktors 10 eine isotherme Umgebung zu schaffen. Zu diesem Zweck kann ein Fluid (z.B. Wasser oder Gas) durch eine Fluidzufuhr 16 in den Fluidraum 14 gelangen. Es ist eine Fluidabfuhr 17 am Fluidraum 14 vorgesehen, um das Fluid abzuführen. Je nach Situation kann gekühlt oder geheizt werden. Die Entleerungsöffnungen 25.1, 25.2 liegen vorzugsweise auch ausserhalb des Fluidraumes 14.

Vorzugsweise kommt bei allen Ausführungsformen eine Steuerung des Reaktors 10 zum Einsatz, die anfangs beim hochfahren" des Reaktors 10 den Fluidraum mit warmem Fluid beaufschlagt, um die Synthesereaktion in Gang zu setzen. Anschliessend wird vorzugsweise ein gekühltes Fluid zugeführt, um Reaktionswärme, die bei der exothermen Synthese entsteht, abzuführen und um so eine isotherme Umgebung zu schaffen. Abhängig von der Kapazität des Reaktors 10 kann Reaktionswärme mittels eines Fluids im Fluidraum 14 zugeführt werden und die Synthesereaktion kann im endothermen Bereich ablaufen.

Vorzugsweise ist der Fluidraum 14 bei allen Ausführungsformen so ausgelegt, dass mindestens die Reaktionsabschnitte der Reaktorröhren 20.n, die mit dem Katalysator 60 gefüllt sind, und die Umlenkelemente 30.k in der isothermen Umgebung liegen.

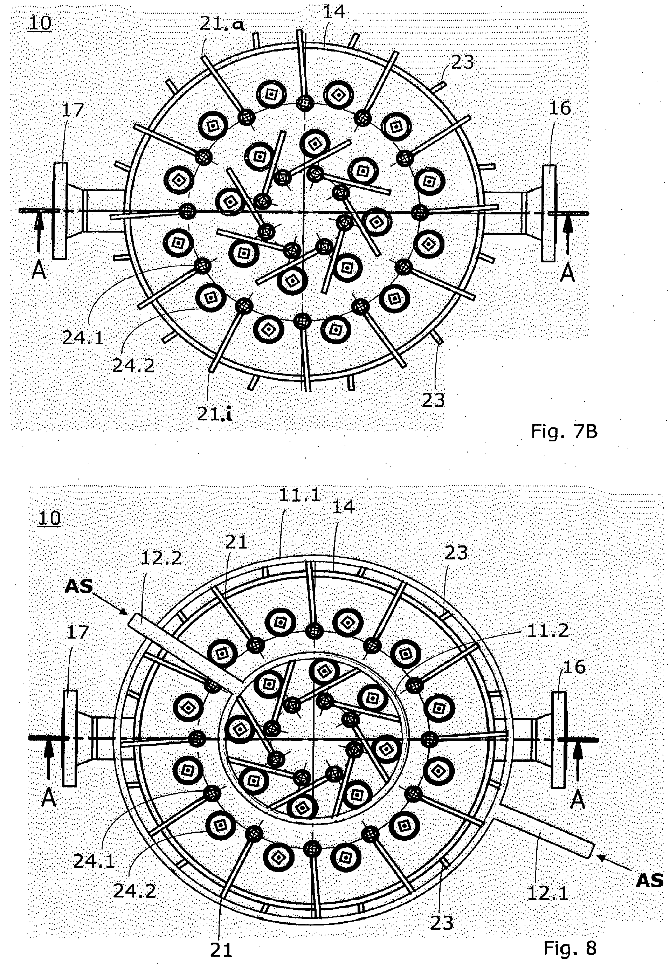

In Fig. 7B ist die Draufsicht des Reaktors 10 gezeigt. Man kann erkennen, dass die Gaseintritte 21.a des äusseren Bündels radial nach außen weisen. Sie enden alle bei einem gemeinsamen ersten Radius. Die Gaseintritte 21.i des inneren Bündels weisen schräg nach aussen und enden alle bei einem gemeinsamen zweiten Radius, der kleiner ist als der erste Radius. Diese Art der Ausrichtung und Anordnung der Gaseintrittel 21a, 21i ermöglicht es, zwei Ringleitungen 11.1 und 11.2 vorzusehen, wie in Fig. 8 schematisch angedeutet. Eine erste obere Ringleitung 11.1 hat einen Radius, der dem ersten Radius so entspricht, dass die Gaseintritte 21.a des äusseren Bündels alle von der ersten oberen Ringleitung 11.1 mit dem Ausgangsstoff AS beschickt werden können. An der ersten oberen Ringleitung 11.1 kann vorzugsweise eine erste Zufuhrleitung 12.1 vorgesehen sein. Eine zweite obere Ringleitung 11.2 hat einen Radius, der dem zweiten Radius entspricht so, dass die Gaseintritte 21.i des inneren Bündels alle von der zweiten oberen Ringleitung 11.2 mit dem Ausgangsstoff AS beschickt werden können. An der zweiten oberen Ringleitung 11.2 kann vorzugsweise eine zweite Zufuhrleitung 12.2 vorgesehen sein. Es können aber auch alle Zufuhrleitungen 21 der inneren 8 Reaktorelemente und der äusseren 12 Reaktorelemente durch eine gemeinsame Ringleitung, die als Sammler oder Verteiler dient, beschickt werden.

In den Figuren 7B und 8 ist zu erkennen, dass Befüllöffnungen 24.1, 24.2 vorzugsweise frei von oben zugänglich sind, um ein einfaches Befüllen und/oder Entlüften und/oder Spülen (z.B. mit inertem Gas) zu ermöglichen.

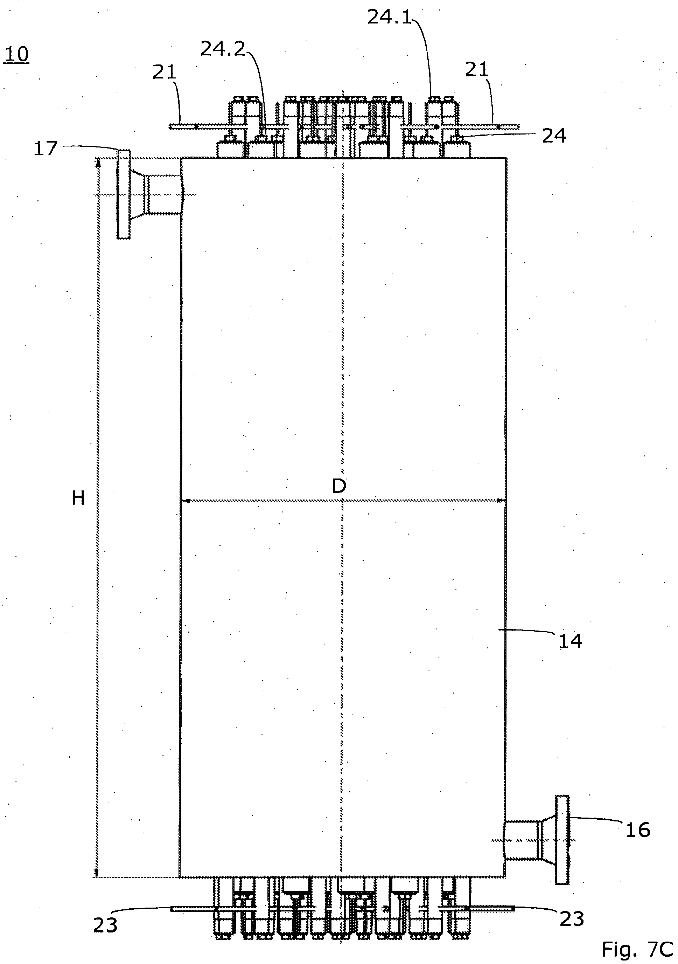

In Fig. 7C ist der Reaktor 10 von aussen gezeigt. Die Bezugszeichen sind dieselben wie in den anderen Figuren. Es wird daher auf die Beschreibung der anderen Figuren verwiesen. Der Fluidraum 14 hat hier einen Hülldurchmesser D, der z.B. ca. 1 m betragen kann. Die Höhe H des Fluidraumes 14 beträgt hier zum Beispiel ca. 2,2 m. Damit lassen sich Reaktorelemente 15.m im Fluidraum 14 unterbringen, die je eine Reaktionsgesamtstrecke von ca. 5,7 m aufweisen. Die Reaktionsgesamtstrecke von ca. 5,7 m setzt sich hier zusammen aus je drei Teilstrecken, da jedes der Reaktorelemente 15.m je n=3 Reaktorröhren 20.1, 20.2 und 20.3 umfasst. Der gegenseitige Rohrabstand AR (siehe Fig. 5E) kann z.B. ca. 60 mm betragen und die einzelnen Reaktorröhren 20.n können z.B. einen Aussendurchmesser von 42 mm und einen Innendurchmesser von 33 mm haben.

Vorzugsweise wird bei allen Ausführungsformen der Erfindung der Ausgangsstoff AS vorgewärmt und/oder mit erhöhtem Druck durch die Zufuhrleitungen 12.1, 12.2 und die Ringleitungen 11.1, 11.2 in die Reaktorelemente 15.m eingebracht. Der Druck und die Temperatur hängen von der Art des Katalysators 60 ab. Vorzugsweise liegt die Temperatur im Bereich zwischen 100 und 350 Grad Celsius. Der Druck liegt typischerweise zwischen 10 und 150 bar.

Es wird als ein wichtiger Vorteil der Erfindung angesehen, dass z.B. bei einem Syntheseverfahren zur Erzeugung von Methanol aus CO₂-Gas 101 und Wasserstoff 103, das 4 Liter Katalysatorvolumen erfordert, um eine gewünschte Produktionsmenge pro Tag zu gewährleisten, die Bauhöhe des Reaktors 10 gegenüber herkömmlichen linearen Reaktoren deutlich reduziert werden kann. Ein herkömmlicher Reaktor hätte z.B. typischerweise eine Gesamtbauhöhe, die im Bereich von ca. 6 m liegt, wohingegen die Höhe H gemäss Erfindung bei dreifach gefalteten Reaktorelementen 15.m ca. 2,2 m beträgt. Die Höhe über alles (d.h. inkl. der Befüllöffnungen 24.1, 24.2, der Entleerungsöffnungen 25.1, 25.2, der Gaseintritte 21 und Produktauslässe 23) beträgt dann ca. 2,6 m.

Es ist ein weiterer Vorteil, dass es durch die "Faltung" der Reaktorröhren 20.n einfacher wird, gleichbleibende (z.B. isotherme) Umgebungsbedingungen für alle Abschnitte der Reaktorelemente 15.m vorzugeben und einzuhalten. Bei einer linearen Reaktorröhre, die zum Beispiel 6 m lang ist, ist es deutlich schwieriger homogene Umgebungsbedingungen vorzugeben und einzuhalten, als bei einer gefalteten Anordnung gemäss Erfindung. Homogene Umgebungsbedingungen sind besonders wichtig, um eine gleichmäßige Umsetzung zu erzielen und um zu verhindern, dass der Katalysator 60 ungleichmäßig beansprucht wird. Ausserdem können z.B. kühlere Bereiche zu einer Unterbrechung oder Beeinflussung der Synthese führen.

Anlagen der vorliegenden Grössen müssen kompakt gebaut werden. Für diesen Fall sind kurze Reaktoren 10, die z.B. in einem Container platziert werden, von Vorteil. Ausserdem entfallen die Stützbleche, die bei längeren Reaktorröhren üblich sind.

Ein Reaktor 10, gemäß Erfindung, kann eine Umsetzungsrate erzielen, die höher ist als bei linearen Röhrenreaktoren.

Die "Faltung" kann unter Umständen die Synthesekinetik ändern. Das wäre z.B. dann der Fall, wenn es in den Umlenkelementen 30.k zu Engpässen oder Blockaden kommen würde. Daher kommt vorzugsweise bei allen Ausführungsformen das inerte Material 61 in den Umlenkelementen 30.k zum Einsatz. Durch den Einsatz des inerten Materials 61 können auch Taupunktverschiebungen vermieden werden. Die Ausgestaltung der Umlenkelemente 30.k, die Ausstattung mit dem inerten Material 61 und deren Anordnung innerhalb des Fluids im Fluidraum 14 verhindern die Methanolkondensation im Inneren der Reaktorelemente 15.m aus dem Reaktionsfluid.

Besonders vorzugsweise kommt bei allen Ausführungsformen hartkeramisches Inertmaterial 61 (vorzugsweise in Kugelform) zum Einsatz.

Besonders vorzugsweise dient bei allen Ausführungsformen das inerte Material 61 in den untenliegenden Umlenkelemente (z.B. 30.1) als Stütz- oder Trägerstruktur unter dem Katalysatorbett (analog z.B. zu einem Kiesbett unter einem Filter).

Besonders vorzugsweise dient bei allen Ausführungsformen das inerte Material 61 in den obenliegenden Umlenkelementen (z.B. 30.2)
- als Schutz gegen strömungsbedingte Beschädigungen bei starker Anströmung, und/oder
- als Rückhalteschicht gegen "tanzenden" Katalysator 60, und/oder
- als Rückhalteschicht bei rückwärtsgerichteten Druckstössen (z.B. bei einem Reaktor-Notaus) oder bei Abwärtsströmungen im Reaktor 10.

Das lose inerte Material 61, das bei allen Ausführungsformen zum Einsatz kommt, hat auch den Vorteil, dass keine festen metallischen Einbauten erforderlich sind. Ausserdem kann das inerte Material 61 problemlos eingefüllt und wieder entnommen werden.

Die Aspekte der verschiedenen Ausführungsformen lassen sich problemlos durch kleinere Anpassungen miteinander kombinieren.

Im Folgenden werden weitere grundlegende Aspekte eines erfindungsgemäßen Verfahrens zum Bereitstellen speicherbarer und transportabler Energieträger beschrieben.

Der Reaktor 10 eignet sich speziell für die Synthese von regenerativem Methanol CH₃OH aus Kohlendioxid CO₂ und Wasserstoff H₂, der über die (endotherme) Elektrolyse von Wasser mit regenerativer elektrischer Energie gemäß Reaktion 1, wie bereits weiter oben erwähnt, erzeugt wird.

H₂O - 286,02 kJ/mol = H₂ + 0,5O₂. (Reaktion 1)

Die exotherme Methanolsynthese (Reaktion 2, wie weiter oben bereits erwähnt) wird durch die Summenformel dargestellt:

CO₂ + 3 H₂ = CH₃OH + H₂O - 49,6 kJ (Methanol gasförmig). (Reaktion 2)

Die eingesetzte regenerative elektrische Energie E1 hat insbesondere im Fall von Wind- und Solarkraft die Nachteile, dass sie relativ teuer ist und nur unregelmäßig und mit zeitlichen Beschränkungen verfügbar ist.

Deshalb wird hier vorgeschlagen, die regenerative Herstellung von Methanol 108 mit regenerativer elektrischer Energie E1 gemäß den Reaktionen 1 und 2 durch eine Methanolsynthese zu ergänzen, die auf der Rohstoffbasis von Erdgas mit dem Hauptbestandteil Methan CH₄, oder auf der Basis anderer Kohlenstoffausgangsmaterialien oder Kohlenwasserstoffausgangsmaterialien basiert. Diese Synthese kann z.B. durch exotherme direkte Oxidation (oxidative Transformation, oxidative Konversion) des Methans nach der Summenformel erfolgen

(CH₄ + 0,50₂ = CH₃OH - 167,5 kJ/mol. (Reaktion 3)

Diese Reaktion 3 wird hier als Direktoxidation bezeichnet. Der erforderliche Sauerstoff kann aus der bereits erwähnten Wasserelektrolyse (Reaktion 1) entnommen werden.

Es kann aber auch der Weg über die partielle Oxidation (siehe Reaktion 4, siehe unten) oder die Reformierung (siehe Reaktionen 6 und/oder 7, siehe unten) (vorzugsweise als autotherme Reformierung) gewählt werden, um über den Weg von Synthesegas (hier im Wesentlichen Kohlenmonoxid und Wasserstoff umfassend) zu einem fossilen Methanolanteil zu kommen. Der entsprechende Sauerstoffanteil kommt typischerweise aus einer Luftzerlegungsanlage.

Neu ist vor allem die Tatsache, dass der (reine) Sauerstoff, respektive das stark sauerstoffhaltige Gas, für Reaktion 3 oder auch für die partielle Oxidation oder die Reformierung direkt oder zwischengespeichert z.B. aus der Hydrolysereaktion (Reaktion 1) oder aus einer anderen Reaktion stammt. Wird der (reine) Sauerstoff, respektive das stark sauerstoffhaltige Gas, aus der Hydrolysereaktion (Reaktion 1) genommen, so reicht gemäß den Massenverhältnissen der Reaktionen 1, 2, und 4 der Sauerstoff aus Reaktion 1 aus, um maximal drei Mal mehr fossiles Methanol mittels Reaktion 3 bereitzustellen als mit Reaktion 2. Bei der Verwendung allen Sauerstoffes aus Reaktion 1 kann das gesamte erzeugte Methanol zu einem Viertel aus der "regenerativen" Reaktion 2, die in einem Reaktor 10 stattfindet, und zu drei Vierteln aus der "fossilen" Reaktion 4 (z.B. der Direktoxidation) stammt.

Durch Verringerung des Methanolanteils aus Reaktion 4 kann der "regenerative" Abteil 108 an der gesamten Methanolproduktion erhöht werden, z.B. auch mit entsprechenden Auswirkungen auf die CO₂-Bilanz der gesamten Methanolproduktion und die spezifischen CO₂-Emissionen bei der Verbrennung des "Gesamt"-Methanols zur Wärmeerzeugung oder als Kraftstoff.

In der Praxis wird vorzugsweise die zur Verfügung stehende regenerative elektrische Energie E1 zur Erzeugung von "regenerativem" Methanol 108 gemäß den Reaktionen 1 und 2 maximal ausgenutzt und der Anteil des nach Reaktion 3 erzeugten "fossilen" Methanols wird nach wirtschaftlichen und ökologischen Zielsetzungen und Randbedingungen bis zum möglichen Maximalwert eingestellt, z.B. nach gewünschter spezifischer CO₂-Emission des "Gesamt"-Methanols bei Verbrennung oder nach dem momentanen Preis und der Verfügbarkeit des Erdgases oder nach der zu erzeugenden "Gesamt"-Methanolmengeoder nach den Preisen der regenerativen und fossilen Methanolanteile.

Es muss betont werden, dass der Reaktor 10 in allen Ausführungsformen natürlich auch für andere Syntheseverfahren eingesetzt werden kann und dass die Synthese mit regenerativer Energie und/oder mit regenerativem Ausgangsstoff AS, aber auch mit fossiler Energie und/oder mit fossilem Ausgangsstoff AS betrieben werden kann.

Besonders vorteilhaft ist, dass das "regenerative" Methanol 108 und das "fossile" Methanol in verschiedenen Reaktoren getrennt anfallen können und entweder getrennt abgegeben werden können oder nach dem Anfall und ggf. Zwischenlagerung in beliebigen Anteilen gemischt werden können, so dass die Silicon-Fire Anlage 700 rein "regeneratives" Methanol 108 und rein "fossiles" Methanol liefern kann, aber auch beliebige Mischungen von beiden, um z.B. als regenerativer Kraftstoff mit zulässigem fossilen Anteil oder zulässiger spezifischer CO₂-Emission vermarktet werden zu können.

Die Reaktionswärmen der exothermen Reaktionen 2 und 3 werden vorzugsweise genutzt, wodurch sich auch die anzurechnenden spezifischen CO₂-Emissionen der verschiedenen Methanolfraktionen verringern würden. Für die Wärmenutzung kommt z.B. unter den Bedingungen des Persischen Golfes sehr vorteilhaft die Beheizung von thermischen Seewasserentsalzungs-Anlagen in Frage, insbesondere während der kälteren Jahreszeit, wenn wegen des wesentlich geringeren Strombedarfes für die Gebäudeklimatisierung Wärmekraftwerke in Teillast gefahren oder sogar abgeschaltet werden und deren Abwärme nicht mehr (ausreichend) für die ihnen angeschlossenen Seewasserentsalzungs-Anlagen zur Verfügung steht. Die Wärme, die zum Beispiel durch das Fluid über den Fluidraum 14 dem Reaktor 10 entzogen wird, kann auch in anderen Anlagenbereichen genutzt werden.

Das Ergebnis der Reaktion 3 kann z.B. durch die Kombination der folgenden großtechnisch erprobten Reaktionen 4 und 5 erreicht werden.

Die partielle Oxidation von Methan:

CH₄ + 0,5O₂ = CO + 2H₂ -36 kJ/mol (Reaktion 4)

und die klassische Methanolsynthese (z.B. 250 bar, 350 °C, mit Chromoxid-Zinkoxid-Katalysator) :

CO + 2H₂ = CH₃OH -128,20 kJ/mol (flüss. Methanol). (Reaktion 5)

Bei der Kombination der Reaktionen 4 und 5 findet quasi auch eine Oxidation des methanhaltigen Gases unter Einsatz von sauerstoffhaltigem Gas statt.

Die Reaktion 5 kann unter Umständen im gleichen Reaktor 10 ablaufen wie die Reaktion 2, evtl. mit Wechsel oder Anpassung der Katalysatorfüllung und der Katalysebedingungen. Es können aber z.B. auch zwei Reaktoren 10, gemäss Erfindung, mit unterschiedlicher Katalysatorfüllung zum Einsatz kommen.

Die Reformierung von Methan mit Wasserdampf (steam reforming) zu Synthesegas läuft endotherm nach der folgenden Reaktion ab:

CH₄ + H₂O (gasf.) = CO + 3H₂ + 206,2 kJ/mol (Reaktion 6)

Die Reformierung von Methan mit Kohlendioxid zu Synthesegas läuft endotherm nach der folgenden Reaktion ab:

CH₄ + CO₂ = 2CO + 2H₂ + 247 kJ/mol (Reaktion 7)

Die drei Reaktionen 4, 6 und 7 können gemeinsam in einem Reaktor 10 bei Temperaturen von ca. 800 - 1000 °C über Katalysatoren ablaufen und so gesteuert werden, dass sie möglichst energieautark ("autotherm") ablaufen und die Reaktionsprodukte ein geeignetes Synthesegas für die klassische Methanolsynthese nach Reaktion 5 ergeben.

Weitere Details sind z.B. der Publikation von Bharadwaj, S.S.; L.D. Schmidt: Catalytic partial oxidation of natural gas to syngas. Fuel Processing Technology 42 (1995), S. 109 - 127 zu entnehmen.

Besonders vorteilhaft ist der Einsatz der Erfindung im Zusammenhang mit einem Verfahren zur Methano synthese, dass bei niedrigen Drücken (z.B. 80 bar) arbeitet.

Das Prinzip der Erfindung lässt sich auch auf Grossanlagen übertragen.

Gemäß Erfindung dient vorzugsweise CO₂ 101 als Ausgangsstoff und Kohlenstofflieferant für die Methanolsynthese im Reaktor 10. Als C0₂-Quellen dienen vorzugsweise: Steam-Reforming-Anlagen, CO₂-Abscheidungsanlagen für Roherdgas, Kalkbrennöfen, Kalzinieranlagen für Soda, Fermentationsanlagen für Bioethanol, Meerwasserentsalzungsanlagen, große Feuerungsanlagen für fossile Brennstoffe (z.B. Kraftwerksfeuerungen) und andere Anlagen oder Verbrennungsprozesse, die relativ große Mengen an CO₂ emittieren.

Die Erfindung ermöglicht es, die erheblichen wirtschaftliche Nachteile bekannter Ansätze zu vermeiden, wenn - wie im Fall der Silicon-Fire Anlage 700 - die instationär anfallende elektrische Solar- und/oder Windenergie direkt in chemische Reaktionsenthalpie umgewandelt und chemisch gebunden gespeichert wird, ohne dass zusätzliche Kapazitäten für Reserveleistungen und/oder Frequenzregelung im Verbundnetz und die dafür erforderlichen Aufwendungen notwendig sind.

Bei photovoltaischer Stromerzeugung mittels einer Photovoltaikanlage 400 ist ein weiterer Vorteil, dass der aus den Solarzellen der Photovoltaikanlage 400 primär anfallende Gleichstrom E1 direkt für den chemischen Prozess (Elektrolyse 105) genutzt werden kann, ohne über Umrichter in Wechselstrom zur Spannungstransformatiön umgewandelt werden zu müssen.

In einer anderen Ausführungsform der Erfindung kann auch ein ATR-Prozess (Autotherme Reformierung) für die Verarbeitung des Ausgangsstoffes AS eingesetzt werden. Bei der autothermen Reformierung wird ein kohlenwasserstoffhaltiges oder ein köhlenstoffhaltiges Ausgangsmaterial in einer Reaktionszone in Gegenwart einer unterstöchiömetrischen Menge an Sauerstoff (z.B. Sauerstoff) oxidiert, die für die vollständige Oxidation nicht ausreicht. Außerdem werden Wasserdampf und/oder Kohlendioxid zugeführt, um auf diesem Weg Synthesegas erzeugen zu können, das im Wesentlichen Kohlenmonoxid und Wasserstoff umfasst. Das Ausgangsmaterial kann Erdgas oder ein anderer Kohlenwasserstoff sein. Es ist auch möglich Kohlen, Öle, Brenngase, Biomassen, Ölsande oder Ölschiefer mit dem ATR-Prozess zu einem geeigneten Synthesegas umzusetzen.

Das Synthesegas aus Kohlenmonoxid und Wasserstoff oder Kohlendioxid und Wasserstoff oder aus Kohlenmonoxid, Kohlendioxid und Wasserstoff kann in einem erfindungsgemässen Reaktor 10 unter Einsatz eines jeweils geeigneten Katalysators 60 zu Methanol 108 umgesetzt werden, wie beschrieben (Kohlenmonoxid und Kohlendioxid werden hier als Kohlenstoffoxide bezeichnet). Je nach Synthesereaktion können z.B. kupfer-basierte Katalysatoren 60 (z.B. CuO-Katalysatoren), oder Zinkoxid-Katalysatoren 60 (z.B. ZnO-Katalysatoren), oder Chromoxid-Zinkoxid-Katalysatoren 60 eingesetzt werden. Auch alle anderen bekannten Katalysatoren eignen sich für den Einsatz in einem Reaktor 10. Besonders geeignet sind Festbett-Katalysatoren oder Flüssigbett-Katalysatoren. Der Katalysator 60 kann auch einen geeigneten Träger (z.B. Karbon, Silikat, Aluminium (z.B. Al₂O₃) oder Keramik umfassen). Statt der erwähnten "metallischen" Katalysatoren 60 kann auch ein organischer Katalysator 60 eingesetzt werden.

Der Katalysator 60 hat vorzugsweise bei allen Ausführungsformen eine Korn-, Kugel- oder Teilchengrösse zwischen 1 und 10 mm. Besonders bevorzugt ist eine Korn-, Kugel- oder Teilchengrösse zwischen 3 und 8 mm. Das inerte Material 61 hat vorzugsweise bei allen Ausführungsformen auch eine Korn-, Kugel- oder Teilchengrösse, die etwa kleiner ist als die Korn-, Kugel- oder Teilchengrösse des Katalysators 60. Besonders bevorzugt sind Ausführungsformen, bei denen die Korngrösse des inerten Materials 61 kleiner ist als die Korngrösse des Katalysators 60, damit der Katalysator 60 nicht in Hohlräume im inerten Material 61 rutscht.

Es wird als ein Vorteil der "Faltung" der Reaktorröhren 20.n und des Einsatzes der Umlenkelemente 30.k angesehen, dass z.B. Temperaturmessstellen an den Umlenkelementen 30.k angeordnet werden können. Damit kann die Synthesereaktion besser überwacht und genauer gesteuert werden.

In einer anderen Ausführungsform der Erfindung wird beim ATR-Prozess Kohlendioxid zugeführt. Das Hinzufügen von CO₂ kann dann von Vorteil sein, wenn die stöchiometrischen Verhältnisse aufgrund der Ausgangsmaterialien für die Synthese von Methanol 108 nicht optimal sind.

In einer besonders bevorzugten Ausführungsform der Erfindung wird das (regenerative und/oder fossile) Methanol als Energieträger zum Lagern und Transportieren eingesetzt.

Kohlendioxid als Kohlenstofflieferant kann gemäß Erfindung auch aus dem Roherdgas entnommen werden, das je nach Erdgasquelle mehr als 10 % Kohlenstoffdioxid-Anteil aufweisen kann. Nach dem Fördern des Roherdgases wird bereits heute typischerweise eine Gaswäsche (mittels Gaswäschetechnologie oder einer anderen Gastrenntechnologie) durchgeführt, um das CO₂ vom eigentlichen Erdgas zu trennen. Meist wird dieses CO₂ in die Atmosphäre emittiert. Gemäß Erfindung kann das CO₂, das in weitgehend reiner Form vorliegt, als Kohlenstofflieferant 101 eingesetzt werden.

Für die Nutzung des Methanols 108 in Verbrennungskraftmaschinen, z.B. auch in kombinierten Gas-/Dampfturbinenanlagen, kann eine katalytische Spaltung (z.B. bei ca. 380 °C) vor der Verbrennung nach der Reaktion 8:

CH₃OH (flüssig) = 2H₂ + CO +128,20 kJ/mol (Reaktion 8)

folgende Vorteile bringen.

Eine normalerweise mit Gas betriebene Verbrennungskraftmaschine braucht nicht auf einen flüssigen Brennstoff umgestellt zu werden. Die Reaktionswärme der endothermen Methanol-Spaltreaktion 8 von 128,20 kJ/mol oder 4006 kJ/kg Methanol kann durch die Abgasabwärme einer Verbrennungskraftmaschine aufgebracht werden, wodurch sich der ursprüngliche Heizwert des Methanols von 19900 kJ/kg um die zugeführte Reaktionswärme um ca. 20 % erhöht. Die Anwendung einer entsprechenden Methanolspaltung für Kraftwerksprozesse wird z.B. beschrieben in Mußenbrock, K: "Möglichkeiten zur Nutzung von Methanol in Kraftwerksprozessen", VGB Kraftwerkstechnik 71 (1991), Heft 8, S. 759 - 764.

### Bezugszeichen:

- Fahrzeugindustrie / Automobilbau: 1
- Kraftwerksbetreiber: 2
- Windfarm: 3

- Reaktor: 10
- gemeinsame Ausgangsstoffzufuhr: 11
- erste obere Ringleitung: 11.1
- zweite obere Ringleitung: 11.2
- Zufuhrleitungen: 12.1, 12.2
- Fluidraum: 14
- Reaktorelemente: 15.m
- Fluidzufuhr: 16
- Fluidabfuhr: 17

- Reaktorröhren: 20.n
- Gaseintritt: 21
- Gaseintritt des äußeren Bündels: 21.a
- Gaseintritt des inneren Bündels: 21.i
- erstes Röhrenende: 22.1
- zweites Röhrenende: 22.2
- Produktauslass: 23
- Befüllöffnung: 24.1, 24.2
- Entleerungsöffnung: 25.2, 25.2

- Umlenkelement: 30.k
- Trennwand: 31

- Katalysator: 60
- Inertes Material: 61

- Silicon-Fire Anlage (aus Parallelanmeldung): 100
- Kohlenstoffdioxid: 101
- Wasser: 102
- Wasserstoff: 103
- Bereitstellen von Kohlenstoffdioxid: 104
- Durchführen einer Elektrolyse: 105
- Abgeben/Bereitstellen von Methanol: 107
- transportabler Energieträger: 108
- (Anlagen-)Steuerung: 110
- Parameterspeicher: 111
- Steuer- oder Signalleitungen: 112, 113, 114
- Solarthermieanlage: 300
- Umwandlung von Wärme in Gleichstrom: 301
- Solaranlage (Photovoltaikanlage): 400
- Verbundnetz: 500
- Umwandlung von Wechselspannung in: 501
- Gleichstrom (Energieversorgungsanlage) siliziumdioxidhaltiges Ausgangsmaterial: 601
- Silizium: 603
- Reduktionsverfahren: 602
- Siliziumdioxid als Rückreaktionsprodukt: 604
- Hydrolyse: 605

- Silicon-Fire Anlage (Erfindung): 700
- Pfeile: 701, 702

- Rohrabstand: AR
- Ausgangsstoff: AS
- Hülldurchmesser: D
- Gleichstromenergie: E1
- Erste Ebene: F1
- zweite Ebene: F2
- Höhe: H
- Effektive Länge: LE
- Inputgrößen: I1, I2, usw.
- Primärenergie: P1, P2

## Patentansprüche

1. Reaktor (10) zur Synthese eines flüssigen Kohlenwasserstoffs, vorzugsweise eines methanolhaltigen Kohlenwasserstoffs (108), unter Einsatz eines Ausgangsstoffes (AS), der ein Gas mit Kohlenstoffanteil und Wasserstoffanteil enthält, wobei der Reaktor (10) mehrere parallel zueinander geschaltete Reaktorelemente (15.m) umfasst und wobei jedes der Reaktorelemente (15.m) einen Gaseintritt (21) an einem ersten Röhrenende (22.1) und einen Produktauslass (23) an einem zweiten Röhrenende (22.2) aufweist, wobei
- jedes der Reaktorelemente (15.m) mindestens eine erste Reaktorröhre (20.1) und eine gegenläufig angeordnete zweite Reaktorröhre (20.n) umfasst,
- jedes der Reaktorelemente (15.m) mindestens ein Umlenkelement (30.k) umfasst, das zwischen der ersten Reaktorröhre (20.1) und der zweiten Reaktorröhre (20.2) sitzt und diese strömungstechnisch verbindet,
- jedes der Reaktorelemente (15.m) mindestens eine Befüllöffnung (24.1, 24.2) zum Einbringen eines Katalysators (60) für die Synthese des flüssigen Kohlenwasserstoffs umfasst,
wobei das Parallelschalten der Reaktorelemente (15.m) dadurch gewährleistet ist, dass die Gaseintritte (21) mehrerer Reaktorelemente (15.m) von einer gemeinsamen Ausgangsstoffzufuhr (12.1, 12.2) mit dem Ausgangsstoff (AS) beschickbar sind, und dass die Produktauslässe (23) mehrerer Reaktorelemente (15.m) strömungstechnisch mit einem nachgeschalteten Anlagenbereich verbindbar sind, **dadurch gekennzeichnet, dass** das Umlenkelement (30.k) mindestens teilweise mit einem inerten Material (61) gefüllt oder ausgestattet ist.

2. Reaktor (10) nach Anspruch 1, **dadurch gekennzeichnet, dass** ein granulares, körniges, kugelförmiges, netzartiges, gitterartiges oder wabenförmiges Material als inertes Material (61) dient.

3. Reaktor (10) nach Anspruch 1 oder 2, **dadurch gekennzeichnet, dass** ein Keramikmaterial als inertes Material (61) dient.

4. Reaktor (10) nach einem der Ansprüche 1 bis 3, **dadurch gekennzeichnet, dass** die gemeinsame Ausgangsstoffzufuhr (12.1, 12.2) in Form einer gemeinsamen Ringleitung (11.1, 11.2) ausgebildet ist.

5. Reaktor (10) nach Anspruch 4, **dadurch gekennzeichnet, dass** er eingangsseitig mindestens eine Ringleitung (11.1, 11.2) umfasst, die als gemeinsame Ausgangsstoffzufuhr (12.1, 12.2) dient.

6. Reaktor (10) nach Anspruch 4 oder 5, **dadurch gekennzeichnet, dass** er ausgangsseitig eine Ringleitung umfasst, die mehrere Reaktorelemente (15.m) strömungstechnisch mit dem nachgeschalteten Anlagenbereich verbindet.

7. Reaktor (10) nach einem der Ansprüche 1 bis 6, **dadurch gekennzeichnet, dass** die Reaktorröhren (20.n) eines Reaktorelements (15.m) so dicht einander sind, dass der gegenseitige lichte Abstand (Aussenwand zu Außenwand) weniger als der Aussendurchmesser eines der Reaktorrohre (20.n) beträgt.

8. Reaktor (10) nach einem der Ansprüche 1 bis 7, **gekennzeichnet durch** ein Reaktorelement (15.m) mit n=2 Reaktorröhren (20.1, 20.2) und einem Umlenkelement (30.1), wobei sowohl der Gaseintritt (21) für den Ausgangsstoff (AS) als auch der Produktauslass (23) am unteren Ende des Reaktorelements (15.m) sitzen.

9. Reaktor (10) nach einem der Ansprüche 1 bis 8, **dadurch gekennzeichnet, dass** jedes der Reaktorelemente (15.m) in Prozessrichtung betrachtet
- den Gaseintritt (21) an dem ersten Röhrenende (22.1),
- eine erste Reaktorröhre (20.1),
- ein erstes Umlenkelement (30.1),
- eine zweite Reaktorröhre (20.2),
- ein zweites Umlenkelement (30.2),
- eine dritte Reaktorröhre (20.3) mit dem dem zweiten Umlenkelement (30.2) gegenüberliegenden, zweiten Röhrenende (22.2), und
- den Produktauslass (23) an dem zweiten Röhrenende (22.2) umfasst.

10. Reaktor (10) nach Anspruch 9, **dadurch gekennzeichnet, dass** das erste Röhrenende (22.1) oben und das zweite Röhrenende (22.2) unten liegt.

11. Reaktor (10) Anspruch 9 oder 10, **dadurch gekennzeichnet, dass** eine Befüllöffnung (24.2) an dem obenliegenden zweiten Umlenkelement (30.2) grösser ist als die obenliegende Befüllöffnung (24.1) am Einlassende der ersten Reaktorröhre (20.1).

12. Reaktor (10) einem der Ansprüche 9 bis 11, **dadurch gekennzeichnet, dass** eine Entleerungsöffnung (25.2) an dem untenliegenden ersten Umlenkelement (30.1) grösser ist als die untenliegende Entleerungsöffnung (25.1) am Auslassende der ersten Reaktorröhre (20.1).

13. Reaktor (10) nach einem der vorhergehenden Ansprüche, **dadurch gekennzeichnet, dass** das es sich bei dem Umlenkelement (30.k) um ein 180-Grad-Umlenkelement (30.k) handelt, das als Umlenkraum ausgebildet ist, der eine 180-Grad Umlenkung eines Fluidstroms im Inneren einer in Prozessrichtung betrachtet vorausgehenden Reaktorröhre (20.1) und einer in Prozessrichtung betrachtet nachgehenden Reaktorröhre (20.2) bewirkt.

14. Reaktor (10) nach einem der Ansprüche 1 bis 13, **dadurch gekennzeichnet, dass** ein Reaktorelement (15.m) eine erste, zweite und dritte Reaktorröhre (20.1, 20.2, 20.3), die im Wesentlichen in einer gemeinsamen Ebene angeordnet sind, umfasst, wobei der Gaseinlass (21) und der Gasauslass (23) schräg in Bezug auf die gemeinsame Ebene angeordnet sind.

15. Reaktor (10) nach einem der Anspruche 1 bis 13, **dadurch gekennzeichnet, dass e**in Reaktorelement (15.m) eine erste, zweite und dritte Reaktoröhre (20-1, 20.2, 20.3) umfasst, wobei die drei Reaktorrhren (20.1, 20.2, 20,3) In einer Winkelanordnung angeordnet sind. wobei die erste. zweite und dritte Reaktorrohre (20.1. 20.2, 20.3) nicht in einer gemeinsamen Ebene tiegen, sondem eine Winkelanordnung aufweisen, bei der eine erste äußere Reaktorröhre [20.1] mit der mittleren Reaktorröbbre (20.2) in einer gemeinsamen ersten Ebene (F1) liegen und wobei die zweite außere Reaktorrahre (20.3) und die mittlere Reaktorröhre (20.2) In einer gemeinsamen zwelten Ebene (F2) liegen.

16. Reaktor (10) nach einem der Ansprüche 1 bis 15, **dadurch gekennzelehnet,** dass er eine Anordnung eines Bündels von Reaktordementen (15.m) aufweist, welche Anordnung ein Innen liegendes Bundel von Reaktorelementen und ein außenllegendes Bundel von Reaktorelementen umfasst, wobei die Gaseinbitte (21.a) ces äußeren Bündelss radial nach außen weisen und bei einem gemeinsamen ersten Radius enden, wobei die Gaseintrite (21.i) des Inneren Bundels scorag nach außen weisen und bei einem gemeinsamen zweiten Radius enden, wobei der zweite Radius kleiner als der erste Radius ist.

17. Reaktor (10) nach Anspruch 16, **gekennzeichnet durch** zwei , Ringleitungen (11.1, 11.2), wobei die erste Ringleitung (11.1) einen Radius aufweist, der dem ersten Radius so entspricht, dass die Gaseintritte (21.a) des äusseren Bundels alle von der ersten oberen Ringleitung (11.1) mit dem Ausgangsstoff (AS) beschickt werden kennen, wobel die zweite Ringleitung (11.2) einen Radius aurweist, der dem zweiten Radius so entspricht, dass die Gaseintritte (21.1) des inneren Bundels alle von der zweiten oberen Rinhgleitung (11,2) mit dem AUsgangsstoft (AS) beschnckt werden können, wobei der zweite Radius kleiner als der erste Radius ist.

18. Reaktor (10) nach einem der vorhergehentden Ansprüche, **dadurch Gekennzeichnet, dass** er einen Fluidraum (14) umfasst, in dossen Inneren die Reaktiorelemente (15.m) samt der Umlenkelemente (30.k) angeordnet sind.

19. Reaktor (10) nach Anspruch 1a, **dadurch gekennzeichnet**, das der Fluidraum (14) mit einem Fluid beschickbar ist, um, je nach Situation, eine isotherme Umgebungsbedingung bereit stellen zu können.

20. Reaktor (10) nach einem der vorhergehenden Arsprüche, dadurch **gekennzelchnet,** dass er mit einer Kohlenstoffoxid-Gas-Quelle, vorzugsweise einer CO₂-Gas-Quelle, und einer Wasserstoffgas-Quelle verbunden ist.

21. Verfahren zum Bereitstellen eines spelcherbaren und transportablen kohlenwasserstoffhaltigen Energieträgers (108) mit den folgenden Schritten:
- Bereitstellen von einem oder mehreren Reaktorelementen (15.m), die jeweils eine Reaktionssfrecke enthältᵣ die sich zusammensetzt aus einer ersten Reaktorröhre (20.1), die mit einem Katalysator (60) bestückt ist, mindestens einem Umlenkelement (30-1), das mit einem inerten Material (61) bestückt ist, und einer weiteren Reaktorröhre (20.2), die mit einem Katalysator (60) bestückt ist, wobei die zweite Reaktorröhre (20.2) gegenläufig zur ersten Reaktorröhre (20.1) angeordnet ist,
- Bereitstellen eines Reaktors (10) mit einem Bündel aus mehreren diesel Reaktorelemente (15.m), die parallel zueinander angeordnet sind,
- Bereitstellen (104) eines Gases mit Kohlenstoffanteil (101) als Kohlenstofflieferant
- bereitstellen eines Wasserstoffanteils (103),
- Bereitstellen eines Ausgangsstoffes (AS), der den Kohlenstoffanteil (101) und den wasserstoffanteil (103) umfasst.
- Einbringen und Vertei len des Ausgangsstoffes (AS) auf das Bündel der mehreren parallel zueinander angeordneten Reaktorelemente (15.m) des Reaktors (10) mittels einer gemeinsamen AusgangsstoFFzufuhr (12.1. 12.2), wobei je ein Anteil des Ausgangsstoffes (AS) In einem der Resktorelemente (15.m) die Reaktionstrecke durchläuft,
- Bereitstelben des Energieträgers (10R) an einem ausganseitigen Ende der keaktorelemente (15.m).

22. Verfahren nach Anspruch 21, **dadurch gekennzeichnet, dass** in einem den Reaktor (10) enthaltenden Flurdraum (14), der mit einem Fluid beschickbar ist, eine isotherme umgeburigsbedingung bereitgestellt wird.

23. Verfahren nach Anspruch 21 oder 22, **Dadurch gekennzeichnet, dass** vor dem Einbringen und Verteilen des Ausgangsstoffes (AS) der Katalysator (60) in den Reaktoröhren (20.1, 20.2) einem Reduktionssornitt zu seiner Aktivierung unterzogen wird, wobei der Katalysator (60) eine Volumenroduktion erfährt, die mindestens telweise durch Nochrutschen, Nachfulten oder Nachgteiten des Inerten Materials (61) kompensiert wird.

24. Verfahren nach einem der Ansprüche 21 bis 23, **dadurch gekennzeichnet, dass** das Bereitstellen des Wasserstoffanteils (103) geschieht, indem entweder
- eine wassererektrolyse (105) ausgeführt wird, blei der direkt aus Wasser oder einer wässerigen Lösung (H₂O; 102) der wassertoffanteil (103) erzeugt wird. oder
- ein Energieträger, der elementares Silizium enthält, einer Oxidationsreaktion unterzogen wird, um den wasserstoffanteil (103) zu erzeugen.

25. Verwendung eines Reaktors (10) nach einem der Anspruche 1 bis 20 zur Synthese von Methanol, wobei vor dem Einleiten der Synthese Abschnitte der Reaktorelemente (15.m) mit dem Katalysator (60) und die umlenkelemente (30.k) mit inertem Material (61) befullt werden.

26. Verwendung nach Anspruch 25, dadurch gekennzelchnet, dass Jedes der Reaktorelemente (15.m) mindestens einen ersten Reaktionsabschnitt, der mit dem Katalysator (60) gefüllt ist, mindestens ein Umlenkelement (30.1). das mit dem inerten Material (61) gefüllt ist, und einen weiteren Reaktionsabschnitt, der mit einem Katalysator (60) gefüllt ist, umfasst.

## Claims

1. A reactor (10) for the synthesis of a liquid hydrocarbon, preferably a methanol-containing hydrocarbon (108), using a starting material (AS), which contains a gas having a carbon share and a hydrogen share, wherein the reactor (10) comprises a plurality of reactor elements (15.m) connected in parallel to one another and wherein each of the reactor elements (15.m) has a gas inlet (21) at a first pipe end (22.1) and a product outlet (23) at a second pipe end (22.2), wherein
- each of the reactor elements (15.m) comprises at least one first reactor pipe (20.1) and one second reactor pipe (20.n) arranged in the opposite direction,
- each of the reactor elements (15.m) comprises at least one redirection element (30.k), which is seated between the first reactor pipe (20.1) and the second reactor pipe (20.2) and connects them with respect to flow,
- each of the reactor elements (15.m) comprises at least one filling opening (24.1, 24.2) for introducing a catalyst (60) for the synthesis of the liquid hydrocarbon,
wherein the connection in parallel of the reactor elements (15.m) is ensured in that the gas inlets (21) of a plurality of reactor elements (15.m) can be charged with the starting material (AS) by a shared starting material feed (12.1, 12.2), and the product outlets (23) of a plurality of reactor elements (15.m) are connectable with respect to flow to a downstream plant region, **characterized in that** the redirection element (30.k) is at least partially filled or equipped with an inert material (61).

2. The reactor (10) according to claim 1, **characterized in that** a granular, grainy, spherical, net-like, latticed, or honeycomb-like material serves as inert material (61).

3. The reactor (10) according to Claim 1 or 2, **characterized in that** a ceramic material serves as inert material (61).

4. The reactor (10) according to one of the claims 1 to 3, **characterized in that** the shared starting material feed (12.1, 12.2) is realized as a shared ring line (11.1, 11.2).

5. The reactor (10) according to claim 4, **characterized in that** it comprises at least one ring line (11.1, 11.2) on the intake side, which is used as shared starting material feed (12.1, 12.2).

6. The reactor (10) according to claim 4 or 5, **characterized in that** it comprises a ring line on the outlet side, which connects a plurality of reactor elements (15.m) with respect to flow to the downstream plant region.

7. The reactor (10) according to one of the claims 1 to 6, **characterized in that** the reactor pipes (20.n) of a reactor element (15.m) are seated closely to one another that the mutual clearance (outer wall to outer wall) is less than the external diameter of one of the reactor pipes (20.n).

8. The reactor (10) according to one of the claims 1 to 7, **characterized by** a reactor element (15.m) with n=2 reactor pipes (20.1, 20.2) and one redirection element (30.1), wherein the gas inlet (21) for the starting material (AS) as well as the product outlet (23) are situated at the bottom end of the reactor element (15.m).

9. The reactor (10) according to one of the claims 1 - 8, **characterized in that** each of the reactor elements (15.m) comprises, viewed in the process direction
- the gas inlet (21) at the first pipe end (22.1),
- a first reactor pipe (20.1),
- a first redirection element (30.1),
- a second reactor pipe (20.2),
- a second redirection element (30.2),
- a third reactor pipe (20.3) with the second pipe end (22.2) being opposite to the second redirection element (30.2), and
- the product outlet (23) at the second pipe end (22.2).

10. The reactor (10) according to claim 9, **characterized in that** the first pipe end (22.1) lies on top and the second pipe end (22.2) lies at the bottom.

11. The reactor (10) according to claim 9 or 10, **characterized in that** a filling opening (24.2) on the upper second redirection element (30.2) is larger than the upper filling opening (24.1) at the inlet end of the first reactor pipe (20.1).

12. The reactor (10) according to one of the claims 9 to 11, **characterized in that** an emptying opening (25.2) on the lower first redirection element (30.1) is larger than the lower emptying opening (25.1) at the outlet end of the first reactor pipe (20.1).

13. The reactor (10) according to one of the preceding claims, **characterized in that** the redirection element (30.k) is a 180° redirection element (30.k), which is implemented as a deflection chamber which causes a 180° deflection of a fluid flow in the interior of a preceding reactor pipe (20.1), viewed in the process direction, and a following reactor pipe (20.2), viewed in the process direction.

14. The reactor (10) according to one of the claims 1 to 13, **characterized in that** a reactor element (15.m) comprises a first, second, and third reactor pipe (20.1, 20.2, 20.3) which are essentially arranged in a common plane, wherein the gas inlet (21) and the product outlet (23) are arranged with an angle with respect to the common plane.

15. The reactor (10) according to one of the claims 1 to 13, **characterized in that** a reactor element (15.m) comprises a first, second, and third reactor pipe (20.1, 20.2, 20.3), wherein the three reactor pipes (20.1, 20.2, 20.3) have an angled arrangement where the first, second, and third reactor pipes (20.1, 20.2, 20.3) are not laying in a common plane but comprise an angled arrangement where a first outer reactor pipe (20.1) lies in a common first plane (F1) with a middle reactor pipe (20.2), and where the second outer reactor pipe (20.3) lies in a common second plane (F2) with the middle reactor pipe (20.2).

16. The reactor (10) according to one of the claims 1 to 15, **characterized in that** it comprises an arrangement of a bundle of reactor elements (15.m), which arrangement comprises an inner bundle of reactor elements and an outer bundle of reactor elements, wherein the gas inlets (21.a) of the outer bundle are pointing radially outward and end at a shared first radius, wherein the gas inlets (21.i) of the inner bundle are pointing diagonally outward and end at a shared second radius, wherein the second radius is smaller than the first radius.

17. The reactor (10) according to claim 16, **characterized by** two ring lines (11.1, 11.2), wherein the first ring line (11.1) has a radius which corresponds to the first radius so that the gas inlets (21.a) of the outer bundle all can be supplied with the starting material (AS) from the first upper ring line (11.1), wherein the second ring line (11.2) has a radius which corresponds to the second radius so that the gas inlets (21.i) all can be supplied with the starting material (AS) from the second upper ring line (11.2), wherein the second radius is smaller than the first radius.

18. The reactor (10) according to one of the preceding claims, **characterized in that** it comprises a fluid chamber (14), in the interior of which the reactor elements (15.m) including the redirection elements (30.k) are arranged.

19. The reactor (10) according to Claim 18, **characterized in that** the fluid chamber (14) can be charged with a fluid, to be able to provide an isothermal environmental condition depending on the situation.

20. The reactor (10) according to one of the preceding claims, **characterized in that** it is connected to a carbon oxide gas source, preferably a CO₂ gas source, and a hydrogen gas source.

21. A method for providing a storable and transportable hydrocarbon-containing energy carrier (108) having the following steps:
- providing one or more reactor elements (15.m), each comprising a reaction path which is put together using a first reactor pipe (20.1), which is equipped with a catalyst (60), at least one redirection element (30.k), which is equipped with an inert material (61), and a further reactor pipe (20.2), which is equipped with a catalyst (60), wherein the second reactor pipe (20.2) is arranged in the opposite direction of the first reactor pipe (20.1),
- providing a reactor (10) with a bundle of several of these reactor elements (15.m), which are arranged in parallel with resperct to each other,
- providing (104) a gas having a carbon share (101) as a carbon supplier,
- providing a hydrogen share (103),
- providing a starting material (AS), which comprises the carbon share (101) and the hydrogen share (103),
- introducing and distributing the starting material (AS) by means of a shared starting material feed (12.1, 12.2) to the bundle of the plurality of the reactor elements (15.m), wherein a respective share of the starting material (AS) runs through the reaction path in each one of the reactor elements (15.m),
- providing the energy carrier (108) at an outlet-side end of the reactor elements (15.m).

22. The method according to claim 21, **characterized in that** an isothermal environmental condition is provided in a fluid chamber (14) which comprises the reactor (10) and which can be charged with a fluid.

23. The method according to claim 21 or 22, **characterized in that,** before the introduction and distribution of the starting material (AS), the catalyst (60) in the reactor pipes (20.1, 20.2) is subjected to a reduction step for its activation, wherein the catalyst (60) experiences a volume reduction, which is at least partially compensated for by slipping, re-filling, or sliding in of the inert material (61).

24. The method according to one of the claims 21 to 23, **characterized in that** the provision of the hydrogen share (103) is performed **in that** either
- a water electrolysis (105) is executed, in which the hydrogen share (103) is produced directly from water or an aqueous solution (H₂O; 102), or
- an energy carrier, which contains elementary silicon, is subjected to an oxidation reaction to produce the hydrogen share (103).

25. A use of a reactor (10) according to one of claims 1 to 20 for the synthesis of methanol, wherein sections of the reactor element (15.m) are filled with the catalyst (60) and the redirection elements (30.k) are filled with inert material (61) before the initiation of the synthesis.

26. The use according to claim 25, **characterized in that** each of the reactor elements (15.m) comprises at least one first reaction section, which is filled with a catalyst (60), at least one redirection element (30.1), which is filled with the inert material (61), and a further reaction section, which is filled with a catalyst (60).

## Revendications

1. Réacteur (10) de synthèse d'un hydrocarbure liquide, de préférence un hydrocarbure contenant du méthanol (108), le matériau de départ utilisé (AS) contenant un gaz qui présente une teneur en carbone et une teneur en hydrogène, le réacteur (10) comprenant plusieurs éléments de réacteur (15.m) montés en parallèle les uns par rapport aux autres, et chacun des éléments de réacteur (15.m) présentant une arrivée de gaz (21) au niveau d'une première extrémité de tube (22.1) et une sortie de produit (23) au niveau d'une deuxième extrémité de tube (22.2) dans lequel :
- chacun des éléments de réacteur (15.m) comprend au moins un premier tube de réacteur (20.1) et un deuxième tube de réacteur (20.n) disposé en sens contraire,
- chacun des éléments de réacteur (15.m) comprend au moins un élément de renvoi (30.k) qui est placé entre le premier tube de réacteur (20.1) et le deuxième tube de réacteur (20.2) et qui les relie fluidiquement,
- chacun des éléments de réacteur (15.m) comprend au moins un orifice de remplissage (24.1, 24.2) pour introduire un catalyseur (60) pour la synthèse de l'hydrocarbure liquide,
le montage parallèle des éléments de réacteur (15.m) étant garanti par le fait que les arrivées de gaz (21) de plusieurs éléments de réacteur (15.m) peuvent être alimentées en matériau de départ (AS) par une arrivée de matériau de départ commune (12.1, 12.2) et que les sorties de produit (23) de plusieurs éléments de réacteur (15.m) peuvent être reliées fluidiquement à une zone de l'installation se situant en aval, **caractérisé en ce que** l'élément de renvoi (30.k) est rempli ou muni au moins partiellement d'un matériau inerte (61).

2. Réacteur (10) selon la revendication 1, **caractérisé en ce qu'**on utilise comme matériau inerte (61) un matériau granulaire, granuleux, sphérique, réticulé, à mailles ou alvéolaire.

3. Réacteur (10) selon la revendication 1 ou 2, **caractérisé en ce qu'**on utilise comme matériau inerte (61) un matériau céramique.

4. Réacteur (10) selon l'une des revendications 1 à 3, **caractérisé en ce que** l'arrivée de matériau de départ commune (12.1, 12.2) est réalisée sous la forme d'une conduite annulaire commune (11.1, 11.2).

5. Réacteur (10) selon la revendication 4, **caractérisé en ce qu'**il comprend côté entrée au moins une conduite annulaire (11.1, 11.2) qui sert d'arrivée de matériau de départ commune (12.1, 12.2).

6. Réacteur (10) selon la revendication 4 ou 5, **caractérisé en ce qu'**il comprend côté sortie une conduite annulaire qui relie fluidiquement plusieurs éléments de réacteur (15.m) à une zone de l'installation située en aval.

7. Réacteur (10) selon l'une des revendications 1 à 6, **caractérisé en ce que** les tubes de réacteur (20.n) d'un élément de réacteur (15.m) sont serrés les uns contre les autres de sorte que l'écart (paroi extérieure contre paroi extérieure) est inférieur au diamètre extérieur de l'un des tubes de réacteur (20.n).

8. Réacteur (10) selon l'une des revendications 1 à 7, **caractérisé par** un élément de réacteur (15.m) avec n=2 tubes de réacteur (20.1, 20.2) et un élément de renvoi (30.1), l'arrivée de gaz (21) pour le matériau de départ (AS) et la sortie de produit (23) étant situées à l'extrémité inférieure de l'élément de réacteur (15.m).

9. Réacteur (10) selon l'une des revendications 1 à 8, **caractérisé en ce que** chacun des éléments de réacteur (15.m), considéré dans la direction de traitement, comprend :
- l'arrivée de gaz (21) au niveau de la première extrémité de tube (22.1),
- un premier tube de réacteur (20.1)
- un premier élément de renvoi (30.1),
- un deuxième tube de réacteur (20.2),
- un deuxième élément de renvoi (30.2),
- un troisième tube de réacteur (20.3) avec la deuxième extrémité de tube (22.2) opposée au deuxième élément de renvoi (30.2), et
- la sortie de produit (23) au niveau de la deuxième extrémité de tube (22.2).

10. Réacteur (10) selon la revendication 9, **caractérisé en ce que** la première extrémité de tube (22.1) est située en haut et la deuxième extrémité de tube (22.2) est située en bas.

11. Réacteur (10) selon la revendication 9 ou 10, **caractérisé en ce que** l'orifice de remplissage (24.2) au niveau du deuxième élément de renvoi du haut (30.2) est plus grand que l'orifice de remplissage supérieur (24.1) au niveau de l'extrémité d'entrée du premier tube de réacteur (20.1).

12. Réacteur (10) selon l'une des revendications 9 à 11, **caractérisé en ce que** l'orifice de vidange (25.2) au niveau du premier élément de renvoi inférieur (30.1) est plus grand que l'orifice de vidange inférieur (25.1) au niveau de l'extrémité de sortie du premier tube de réacteur (20.1).

13. Réacteur (10) selon l'une des revendications précédentes, **caractérisé en ce que** l'élément de renvoi (30.k) est un élément de renvoi à 180 degrés (30.k) qui est conçu comme une chambre de déviation qui opère un renvoi de 180 degrés d'une circulation de fluide à l'intérieur d'un tube de réacteur (20.1) précédent vu dans le sens de traitement et à l'intérieur d'un tube de réacteur suivant (20.2) vu dans le sens de traitement.

14. Réacteur (10) selon l'une des revendications 1 à 13, **caractérisé en ce qu'**un élément de réacteur (15.m) comprend un premier, un deuxième et un troisième tube de réacteur (20.1, 20.2, 20.3) qui sont agencés essentiellement dans un plan commun, l'arrivée de gaz (21) et la sortie de gaz (23) étant agencées de façon oblique par rapport au plan commun.

15. Réacteur (10) selon l'une des revendications 1 à 13, **caractérisé en ce qu'**un élément de réacteur (15.m) comprend un premier, un deuxième et un troisième tube de réacteur (20.1, 20.2, 20.3), les trois tubes de réacteur (20.1, 20.2, 20.3) étant agencés suivant une disposition angulaire, le premier, le deuxième et le troisième tube de réacteur (20.1, 20.2, 20.3) n'étant pas situés dans un plan commun, mais présentent un agencement angulaire, dans lequel un premier tube de réacteur extérieur (20.1) est situé dans un plan commun (F1) avec le tube de réacteur du milieu (20.2) et dans lequel le deuxième tube de réacteur extérieur (20.3) et le tube de réacteur du milieu (20.2) sont situés dans un deuxième plan commun (F2).

16. Réacteur (10) selon l'une des revendications 1 à 15, **caractérisé en ce qu'**il présente un agencement d'un faisceau d'éléments de réacteur (15.m), lequel agencement comprend un faisceau intérieur d'éléments de réacteur et un faisceau extérieur d'éléments de réacteur, les arrivées de gaz (21.a) du faisceau extérieur étant agencées radialement vers l'extérieur et se terminant au niveau d'un premier rayon commun, les arrivées de gaz (21.i) du faisceau intérieur étant orientées inclinées vers l'extérieur et se terminant au niveau d'un deuxième rayon commun, le deuxième rayon étant plus petit que le premier rayon.

17. Réacteur (10) selon la revendication 16, **caractérisé par** deux conduites annulaires (11.1, 11.2), la première conduite annulaire (11.1) présentant un rayon qui correspond au premier rayon, de sorte que les arrivées de gaz (21.a) du faisceau extérieur peuvent toutes être alimentées en matériau de départ (AS) par la première conduite annulaire supérieure (11.1), la deuxième conduite annulaire (11.2) présentant un rayon qui correspond au deuxième rayon de sorte que les arrivées de gaz (21.i) du faisceau intérieur peuvent toutes être alimentées en matériau de départ (AS) par la deuxième conduite annulaire supérieure (11.2), le deuxième rayon étant plus petit que le premier rayon.

18. Réacteur (10) selon l'une des revendications précédentes, **caractérisé en ce qu'**il comprend un espace fluidique (14) à l'intérieur duquel sont agencés les éléments de réacteur (15.m) avec les éléments de renvoi (30.k).

19. Réacteur (10) selon la revendication 18, **caractérisé en ce que** l'espace fluidique (14) peut être alimenté en fluide pour, selon la situation, pouvoir préparer une condition d'environnement isotherme.

20. Réacteur (10) selon l'une des revendications précédentes, **caractérisé en ce qu'**il est raccordé à une source de gaz d'oxyde de carbone, de préférence une source de gaz CO₂, et à une source de gaz hydrogène.

21. Procédé pour mettre à disposition une source d'énergie (108) contenant de l'hydrocarbure stockable et transportable, comprenant les étapes consistant à :
- préparer un ou plusieurs éléments de réacteur (15.m) comprenant respectivement un tronçon de réaction qui se compose d'un premier tube de réacteur (20.1) qui est chargé d'un catalyseur (60), au moins un élément de renvoi (30.1) qui est chargé d'un matériau inerte (61) et un autre tube de réacteur (20.2) qui est chargé d'un catalyseur (60), le deuxième tube de réacteur (20.2) étant agencé en sens contraire par rapport au premier tube de réacteur (20.1),
- préparer un réacteur (10) avec un faisceau de plusieurs de ces éléments de réacteur (15.m) qui sont agencés parallèlement les uns aux autres,
- préparer (104) un gaz avec une teneur en carbone (101) comme source de carbone,
- préparer une teneur en hydrogène (103),
- préparer un matériau de départ (AS) qui comprend la teneur en carbone (101) et la teneur en hydrogène (103),
- amener et répartir le matériau de départ (AS) sur le faisceau des plusieurs éléments de réacteur (15.m) agencés parallèlement les uns aux autres du réacteur (10) au moyen d'une arrivée de matériau de départ commune (12.1, 12.2), une fraction du matériau de départ (AS) parcourant le tronçon de réaction dans l'un des éléments de réacteur (15.m),
- préparer la source d'énergie (108) au niveau d'une extrémité côté sortie des éléments de réacteur (15.m).

22. Procédé selon la revendication 21, **caractérisé en ce qu'**il est préparé dans un espace fluidique (14) contenant le réacteur (10), lequel peut être alimenté en fluide, une condition d'environnement isotherme.

23. Procédé selon la revendication 21 ou 22, **caractérisé en ce que**, avant d'amener et de répartir le matériau de départ (AS) du catalyseur (60) dans les tubes de réacteur (20.1, 20.2), ce dernier est soumis à une étape de réduction pour l'activer, le catalyseur (60) subissant une réduction de volume qui est compensée au moins partiellement par l'écoulement, le remplissage ou le glissement du matériau inerte (61).

24. Procédé selon l'une des revendications 21 à 23, **caractérisé en ce que** la préparation de la teneur en hydrogène (103) se fait
- soit par électrolyse de l'eau (105), au cours de laquelle la teneur en hydrogène (103) est produite directement à partir de l'eau ou à partir d'une solution aqueuse (H₂O ; 102),
- soit par une réaction d'oxydation à laquelle est soumise la source d'énergie qui contient du silicium élémentaire pour produire la teneur en hydrogène (103).

25. Utilisation d'un réacteur (10) selon l'une des revendications 1 à 20 pour faire la synthèse de méthanol, dans laquelle, avant de commencer la synthèse, des sections des éléments de réacteur (15.m) sont remplies de catalyseur (60) et les éléments de renvoi (30.k) sont remplis de matériau inerte (61).

26. Utilisation selon la revendication 25, **caractérisée en ce que** chacun des éléments de réacteur (15.m) comprend au moins une première section de réaction qui est remplie de catalyseur (60), au moins un élément de renvoi (30.1) qui est rempli de matériau inerte (61) et une autre section de réaction qui est remplie d'un catalyseur (60).
